# EUROPEAN PATENT APPLICATION

(11) **EP 4 019 017 A1**
(43) Date of publication of application: **29.06.2022**
(21) Application number: 21217636.6
(22) Date of filing: 23.12.2021
(51) Int. Cl.: A61K 31/4045, A61K 31/405, A61K 31/4525, A61K 31/59, A61K 31/593, A61K 31/675, A61K 36/00, A61K 36/35, A61K 36/36, A61K 36/48, A61K 36/53, A61K 9/00, A61P 25/20, A61K 31/165, A61K 31/197

(54) **COMPOSITION COMPRISING MELATONIN, PIPERINE AND VITAMIN D, CONTROLLED-RELEASE FORMULATION AND USE THEREOF FOR THE TREATMENT OF SLEEP DISORDERS**

(30) Priority: 23.12.2020 IT 202000032345
(71) Applicant: Uriach Consumer Healthcare, S.L., 08184 PALAU-SOLITÀ I PLEGAMANS (ES)
(72) Inventor: Fortuny Solà, Ana, 08184 Palau-solità i Plegamans - Barcelona (ES); Raventós Colomer, Rosa Maria, 08184 Palau-solità i Plegamans - Barcelona (ES)
(74) Representative: Hoffmann Eitle

(57) **Abstract**

The present invention related to a composition comprising at least melatonin, piperine and vitamin D and a controlled-release formulation thereof for the treatment of sleep disorders. The controlled-release formulation of the present invention comprises a fast release composition to promote a rapid sleep onset, a medium release composition that contributes to a restorative sleep, and a prolonged release composition that provides a good quality sleep during the night.

## Description

The present invention refers to a composition comprising melatonin, piperine and/or capsaicin or a capsaicin analogue (preferably phenylcapsaicin), and vitamin D. Further, the present invention relates to a controlled-release formulation comprising said composition for the treatment of sleep disorders. The controlled release formulation of the present invention may comprise a fast release composition comprising melatonin, piperine and vitamin D to decrease the time to fall asleep (rapid sleep onset), a medium release composition that induces relaxation and provides a restorative sleep, and a prolonged release composition that contributes to a good quality sleep during the night.

Sleep is defined as a state of rest opposed to wakefulness, a state of physical and mental rest characterised by the temporary detachment of consciousness and will, by the slowing down of neurovegetative functions and the partial interruption of the sensory-motor relations of the subject with the environment. An appropriate sleep is biologically necessary to support life. The physical and mental health condition of the person depends on the quality and duration of sleep.

The International classification of sleep disorders (ICSD 2005) gathers more than 90 of them. The most common sleep diseases can be distinguished into dyssomnias, parasomnias and breathing-related sleep disorders.

Dyssomnias are disorders that hinder the individual from falling asleep or cause early awakening and they are characterised by dysfunctional quality, quantity or times of sleep. Particularly known among dyssomnias is insomnia, characterised by inability to fall asleep. Insomnia is associated to poor daytime functioning, with symptoms such as tiredness, irritability, learning difficulty, lack of memory consolidation and marked loss of interest to carry out daily chores. Insomnia lasting for more than a few nights in a row can become "chronic" and cause a sleep deficit which is extremely harmful to the health of the insomniac. Parasomnias usually occur in the non-REM sleep stage and they are mainly related to psychological sleep and dream disorders.

Although there is no unique solution for sleep cycle disorders/dysfunctions, there is a variety of possible remedies available on the market, some from natural sources and others from pharmaceutical research. Phytocompounds, botanical extracts and melatonin are examples of "natural" remedies. Barbiturates, benzodiazepines, neuroleptics, non-benzodiazepine hypnotics and pyrazolopyrimidines are the categories of psychopharmaceuticals (or hypnotic drugs) that are often administered for sleep disorders.

Hypnotic drugs are effective but can lead to dependency and are associated with adverse effects, such as sedation and errors in completing regular tasks the day after use, as well as potential nausea, vomiting, confusion, or, in the case of barbiturates, intoxication and cardiorespiratory depression. Moreover, after a period of administration, hypnotic drugs can lead to a loss or reduction of efficacy (time-induced tolerance).

Conversely, melatonin and/or botanical extracts may be more tolerated than hypnotic drugs, but they show poor or partial efficacy, such as no-long efficacy (as number of hours of sleep) or treatment of the difficulty to fall asleep without providing a gut quality and restorative sleep.

WO 2017/109300 discloses a composition comprising, as active substances, at least melatonin, passion flower, chamomile and lemon balm for use in the treatment of sleep disorders.

The technical problem that the present invention addresses is to provide innovative and effective formulations and compositions for use in a method to treat sleep disorders and symptoms thereof, which are free of side effects and free of the drawbacks of the product present on the market for aiding sleep (both synthetic compounds and natural occurring compounds).

As a result of an extensive research programme, the technical solution that the present invention provides to the above-mentioned technical problem is represented by a composition, preferably for oral administration, (pharmaceutical composition, composition for medical device, composition for dietary supplement or food for special medical purpose, briefly "composition of the invention") comprising or, alternatively, consisting of at least melatonin, piperine and/or capsaicin or a capsaicin analogue (preferably phenylcapsaicin), vitamin D (preferably vitamin D3) and, optionally, vitamin B6, and, optionally, Zea *mays* extract and/or capsaicin or a capsaicin analogue (preferably phenylcapsaicin). Additionally, the composition of the present invention may comprise further active ingredients such as *Griffonia* extract (or another source of 5-hydroxytryptophan, 5-HTP) and/or botanic extracts, for example extract of *Valeriana officinalis,* extract of *Melissa officinalis,* extract of *Passiflora incarnata* and/or extract of *Eschscholzia californica,* and/or Zea *mays* extract, and/or gamma-aminobutyric acid (GABA) or a salt or an ester thereof.

Moreover, the technical solution that the present invention provides to the above-mentioned technical problem is represented by a formulation for oral administration (pharmaceutical formulation, formulation for medical device, formulation for dietary supplement or food for special medical purpose, briefly "formulation of the invention") comprising a fast release composition to provide a rapid sleep onset comprising melatonin, piperine and/or capsaicin or a capsaicin analogue (preferably phenylcapsaicin), vitamin D (preferably vitamin D3) and, optionally, vitamin B6, a medium release composition to provide a restorative sleep comprising botanic extracts, such as extract of *Valeriana officinalis* and/or extract of *Melissa officinalis,* and a prolonged release composition to provide a good quality sleep comprising *Griffonia* extract or another source of 5-hydroxytryptophan (5-HTP), or Zea *mays* extract, or gamma-aminobutyric acid (GABA) or a salt or an ester thereof; optionally, extract of *Passiflora incarnata* and/or extract of *Eschscholzia californica.*

The administration of the composition according to the present invention (that comprises at least melatonin, piperine and/or capsaicin or a capsaicin analogue (preferably phenylcapsaicin), and a vitamin D (or D3)) is able to promote a rapid sleep onset. Moreover, the composition according to the present invention is also able to promote a restorative and good quality sleep (without nocturnal awakenings) and makes it easy to wake up in the morning in a refreshing way (with a feel of restoration).

The controlled-release formulation according to the present invention, when administered in the evening, promotes a rapid sleep onset, a restorative and good quality sleep (without nocturnal awakenings), and makes it easy to wake up in the morning in a refreshing way (with a feel of restoration).

The effectiveness of the controlled-release formulations of the present invention are not only due to the selection of the specific active ingredients comprised in the formulation itself, but also to the tailored formulation for each active ingredient in a controlled-release composition that optimizes its pharmacokinetic and maximize its effectiveness (action at the time in point as needed).

In particular, melatonin, piperine and/or capsaicin or a capsaicin analogue (preferably phenylcapsaicin), vitamin D and, optionally, vitamin B6, and optionally Zea *mays* extract and/or capsaicin or a capsaicin analogue (preferably phenylcapsaicin), are formulated in a fast release composition as they promote a rapid sleep onset and supplement the subject with fundamental active ingredients for aiding sleep. Melatonin is a hormone produced by the pineal gland, a small organ in the brain which responds to light. Its production is stimulated by darkness and suppressed by light, and it is the key for helping the mind to recognize when it is time to sleep and to wake up.

Piperidine increases the absorption and bioavailability of the rest of the ingredients contained in the composition or controlled-release formulation of the present invention.

Vitamin B6 (pyridoxal phosphate) is involved in the L-tryptophan metabolism, and the supplementation of vitamin B6 corrects most drug-induced disorders of tryptophan metabolism.

Being deficient in vitamin D, particularly vitamin D3, can lead to a host of sleep issues, including sleep disruption, insomnia, and overall poor sleep quality.

Botanical extracts that promote relaxation, such as *Valeriana officinalis* and/or *Melissa officinalis,* preferably formulated in a medium release composition (or composition layer), contribute to a restorative sleep during the main hours of a night sleep.

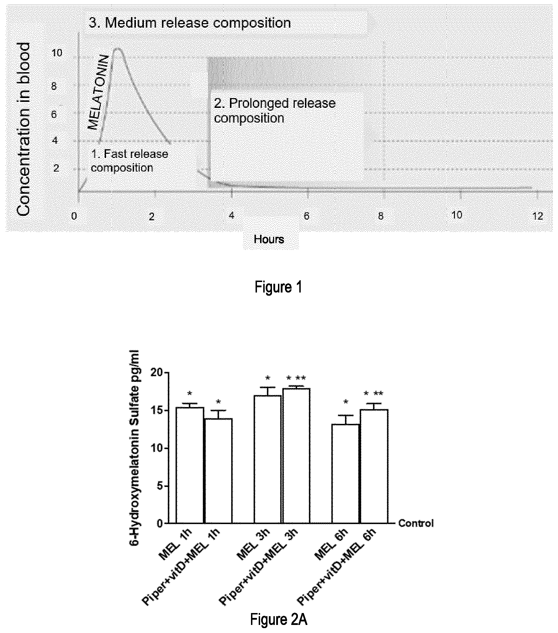

5-Hydroxytryptophan (as such or as extract of *Griffonia*) and, optionally, extract of *Passiflora incarnata* and/or *Eschscholzia californica,* or Zea *mays* extract, or Zea *mays* extract and extract of *Passiflora incarnata* and extract of *Eschscholzia californica,* or gamma-aminobutyric acid (GABA) or a salt or an ester thereof, preferably formulated in a prolonged release composition (or composition layer), maintain the treated subject asleep for 3-4 hours after the administration of the composition or the controlled-release formulation of the present invention, when the blood bioavailability of the administered melatonin (for example within the fast release composition layer) is at minimum level (Figure 1).

5-Hydroxytryptophan is a chemical precursor in the biosynthesis of the neurotransmitter serotonin (5-hydroxytryptamine; 5-HT), and serotonin is the chemical precursor in the biosynthesis of melatonin. Serotonin and melatonin are necessary neurotransmitters for a regular sleep: melatonin helps regulate the body's circadian rhythm, while serotonin communicates a need for sleep to the brain.

*Passiflora incarnata* (passionflower or maypop) aids to relieve insomnia and anxiety. It appears that *Passiflora* boosts the level of gamma-aminobutyric acid (GABA) in the brain and GABA lowers brain activity, helping relaxing and sleep gut quality.

*Eschscholzia californica* (*California poppy*) shows anxiolytic, analgesic, sedative and/or hypnotic effects (dependent from the dosage), mainly as a consequence of its interaction with benzodiazepine receptors.

Moreover, the compositions and controlled-release formulations of the invention are basically free of side effects, particularly free of the typical side effects of hypnotic drugs currently on the market. The compositions and controlled-release formulations are also well tolerated by the subject in need thereof, and they may be used by a large population of subjects, from adults up to senility stage.

The compositions and controlled-release formulations of the present invention are easy to produce and economically advantageous.

These aims, and others which will become clear from the following detailed description, are achieved by the formulations and compositions of the present invention due to the technical characteristics disclosed in the description and claimed in the appended claims.

### BRIEF DESCRIPTION OF THE FIGURES

Figure 1 describes schematically the release kinetics of melatonin and the time of disintegration of the three controlled-release compositions comprised in the formulation of the present invention.
Figures 2A (plasmatic pass) and 2B (hepatic pass) describes the concentration of melatonin metabolite (6-hydroxymelatonin sulphate) detected after plasma pass and liver pass (* p<0.05 vs control; ** p<0.05 vs melatonin).
Figure 3 shows the vitality of hepatic cell treated with melatonin alone or a combination of piperine, vitamin D3 and melatonin (* p<0.05 vs control; ** p<0.05 vs melatonin).
Figures 4A and 4B show the activity of CYP1A2 and CYP3A4 with melatonin alone or a combination of piperine, vitamin D3 and melatonin (* p<0.05 vs control; ** p<0.05 vs melatonin).
Figure 5 shows the activity (%) of the melatonin receptor vs control for melatonin alone and a combination of piperine, vitamin D3 and melatonin (* p<0.05 vs control; ** p<0.05 vs melatonin)

### SUMMARY OF THE INVENTION

It is an object of the present invention a composition (briefly, the composition of the invention) comprising or, alternatively, consisting of a mixture comprising at least melatonin, piperine and/or capsaicin or a capsaicin analogue (preferably phenylcapsaicin), and a vitamin D (preferably D3).

It is an object of the present invention a controlled-release formulation for oral administration (briefly, the controlled-release formulation of the invention or formulation of the invention) comprising: a fast release composition comprising melatonin, piperine and/or capsaicin or a capsaicin analogue (preferably phenylcapsaicin), vitamin D (preferably D3) and, optionally, vitamin B6; a medium release composition comprising *Valeriana officinalis* extract and/or *Melissa officinalis* extract; and a prolonged release composition comprising *Griffonia* extract or 5-hydroxytryptophan (5-HTP), or *Zea mays* extract, or gamma-aminobutyric acid (GABA) or a salt or an ester thereof, and, optionally, *Passiflora incarnata* extract and/or *Eschscholzia californica* extract.

It is an object of the present invention to provide the compositions and the controlled-release formulations according to the present invention for use as a medicament.

It is an object of the present invention the compositions or controlled-release formulations of the invention for use in a method of a preventive and/or curative treatment of sleep disorders and symptoms thereof.

It is an object of the present invention a method to treat sleep disorders, the method comprising the administration of an effective therapeutic quantity of a composition or controlled-release formulation according to the present invention to a subject in need thereof.

It is an object of the present invention a non-therapeutic use of a composition or controlled-release formulation according to the present invention for sleep related problems in a healthy subject.

### DETAILED DESCRIPTION OF THE INVENTION

It is an object of the present invention a composition comprising a mixture (active ingredients) comprising or, alternatively, consisting of melatonin, piperine (or *Piper nigrum* extract), vitamin D (preferably D3) and, optionally, vitamin B6.

The composition of the present invention may further comprise at least one additive and/or excipient of pharmaceutical or food grade.

The composition of the present invention preferably comprises a mixture (active ingredients) comprising or, alternatively, consisting of melatonin, piperine, vitamin D3, vitamin B6, and optionally Zea *mays* extract and/or capsaicin or a capsaicin analogue (preferably phenylcapsaicin).

According to an embodiment of the composition of the present invention, the mixture of active ingredients further comprises an extract of *Griffonia* (or *Griffonia simplicifolia*) or 5-hydroxytryptophan (5-HTP) or another source of 5-HTP. For example, the composition of the present invention comprises a mixture of active ingredients comprising or, alternatively, consisting of melatonin, piperine and/or capsaicin or a capsaicin analogue (preferably phenylcapsaicin), vitamin D (or D3), extract of *Griffonia* (or 5-HTP) and, optionally, vitamin B6.

According to another embodiment of the composition of the present invention, the mixture of active ingredients further comprises a vitamin B6, and optionally Zea *mays* extract and/or capsaicin or a capsaicin analogue (preferably phenylcapsaicin), in addition to said vitamin D; preferably, said vitamin D is a vitamin D3; more preferably, said mixture comprises a melatonin, a piperine, a vitamin D3, a vitamin B6 and, optionally, Zea *mays* extract and/or capsaicin or a capsaicin analogue (preferably phenylcapsaicin).

Preferably, said mixture further comprises at least one active ingredient selected in the group consisting of: an extract of *Valeriana officinalis,* extract of *Melissa officinalis,* extract of *Passiflora,* extract of *Eschscholzia californica,* Zea *mays* extract, and gamma-aminobutyric acid (GABA) or a salt or an ester thereof. More preferably, said mixture comprises or, alternatively, consists of:
(A) melatonin, piperine, vitamin D3, vitamin B6, extract of *Griffonia,* extract of *Passiflora,* extract of *Eschscholzia californica,* extract of *Valeriana officinalis,* and extract of *Melissa officinalis;* or
(B) melatonin, piperine, vitamin D3, vitamin B6, Zea *mays* extract, extract of *Valeriana officinalis,* and extract of *Melissa officinalis;* or
(C) melatonin, Zea *mays* extract, piperine, vitamin D3, vitamin B6, extract of *Passiflora,* extract of *Eschscholzia californica,* extract of *Valeriana officinalis,* and extract of *Melissa officinalis;* or
(D) melatonin, piperine, vitamin D3, vitamin B6, gamma-aminobutyric acid (GABA) or a salt or an ester thereof, extract of *Passiflora,* extract of *Eschscholzia californica,* extract of *Valeriana officinalis,* and extract of *Melissa officinalis.*

In the above embodiments from (A) to (D), the component piperine can be present in addition to a capsaicin or a capsaicin analogue (preferably phenylcapsaicin) or, alternatively, the component piperine can be substituted by a capsaicin or a capsaicin analogue (preferably phenylcapsaicin).

According to a still other embodiment of the composition of the present invention, the mixture of active ingredients (i.e. melatonin, piperine and/or capsaicin or a capsaicin analogue (preferably phenylcapsaicin), vitamin D (or D3), and, optionally, vitamin B6 and/or extract of *Griffonia* (or 5-HTP)) further comprises at least one active ingredient selected in the group consisting of: an extract of *Valeriana officinalis,* extract of *Melissa officinalis,* extract of *Passiflora,* extract of *Eschscholzia californica,* and mixture thereof.

According to a preferred embodiment of the composition of the present invention, the mixture of active ingredients comprises or, alternatively, consists of: melatonin, piperine and/or capsaicin or a capsaicin analogue (preferably phenylcapsaicin), vitamin D (or D3), vitamin B6, extract of *Griffonia,* extract of *Passiflora,* extract of *Eschscholzia californica,* extract of *Valeriana officinalis,* and extract of *Melissa officinalis.*

It is an object of the present invention a controlled-release formulation for oral administration comprising controlled-release compositions as follows:
- a fast release composition comprising as active ingredients melatonin, piperine (or *Piper nigrum* extract) and/or capsaicin or a capsaicin analogue (preferably phenylcapsaicin), vitamin D (preferably D3),
- a medium release composition comprising an extract of *Valeriana officinalis* and/or an extract of *Melissa officinalis,* and
- a prolonged release composition comprising as active ingredient an extract of *Griffonia* (or *Griffonia simplicifolia*) or 5-hydroxytryptophan (5-HTP), or Zea *mays* extract, or gamma-aminobutyric acid (GABA) or a salt or an ester thereof,
   wherein the fast release composition is degraded *in vivo* within a period of time comprised from about 0 minute to about 20 minutes, preferably from about 0 minute to about 5 minutes, after administration (e.g. 0-5 minutes, 0-10 minutes, 0-15 minutes),
   wherein the medium release composition is degraded *in vivo* within a period of time comprised from about 0 minute to about 2.5 hours, preferably from about 0 minute to 2 hours, after administration (e.g. from 0 to 1 hour, from 0 to 1.5 hour),
   wherein the prolonged release composition is degraded *in vivo* within a period of time comprised from about 1.5 hours to about 5 hours, preferably from 2 hours to 4 hours, after administration (e.g. from 1.5 or 2 to 2.5 hours, from 1.5 or 2 to 3 hours, from 1.5 or 2 to 3.5 hours).

In a preferred embodiment, the controlled-release formulation according to the present invention is a monolithic system (for example a tablet) comprising said controlled-release compositions of the present invention (i.e. fast, medium and prolonged release compositions), wherein the controlled-release compositions are compressed composition.

In a preferred embodiment, the compressed compositions are in stacked arrangement (for example a sandwich arrangement). Preferably, in the stacked or sandwich arrangement the fast release compressed composition and the medium release compressed composition are in an external position and the prolonged release compressed composition is in between to the fast release and the medium release compositions. Alternatively, in the stacked or sandwich arrangement the fast release compressed composition and the prolonged release compressed composition are in an external position and the medium release compressed composition is in between to the fast release and the prolonged release compositions. Said stacked arrangement or sandwich arrangement may comprise the controlled-release compositions in any sequence (for example, starting from the first external part of the monolithic system (e.g. tablet): prolonged-medium-fast, prolonged-fast-medium, or fast-prolonged-medium).

In an alternative embodiment, the controlled-release compositions (for example compressed compositions) are in a core-shell arrangement, wherein the prolonged, medium and fast release compositions are in a concentric arrangement (e.g. wrap layers). For example, the prolonged release composition may be the core portion, the medium release may be the first shell that coats the core and the fast release composition may be the second shell that coats the first shell. Said core-shell arrangement may comprise the controlled-release composition in any sequence (for example, starting from the core: prolonged-medium-fast, prolonged-fast-medium, fast-prolonged-medium, or fast-medium-prolonged).

The controlled-release formulation of the present invention may comprise the compressed compositions in any possible arrangement.

The controlled-release formulation of the present invention may have any plausible shape (for example, circular tablet, square tablet, rectangular tablet, spherical tablet) and any size.

The controlled-release compositions (or compressed composition) comprised in the controlled-release formulation of the present invention may have any plausible shape (for example, circular, square, rectangular tablet, spherical tablet) and any size. Different controlled-release compositions comprised in the same controlled-release formulation may have different shape and/or size.

According to an embodiment of the controlled-release formulation of the present invention (comprising the fast-, medium- and prolonged- release compositions), the prolonged release composition further comprises at least one active ingredient selected in the group comprising or, alternatively, consisting of: an extract of *Passiflora,* an extract of *Eschscholzia californica* and/or Zea *mays* extract and/or gamma-aminobutyric acid (GABA) or a salt or an ester thereof, and mixtures thereof; preferably, the prolonged release composition comprises as active ingredient extract of *Griffonia* (or *Griffonia simplicifolia*) or 5-hydroxytryptophan (5-HTP) and *Passiflora incarnata* extract and extract of *Eschscholzia californica,* or an extract of *Passiflora incarnata* and an extract of *Eschscholzia californica,* or Zea *mays* extract, or *Passiflora incarnata* and an extract of *Eschscholzia californica* and Zea *mays* extract, or *Passiflora incarnata* and an extract of *Eschscholzia californica* and gamma-aminobutyric acid (GABA) or a salt or an ester thereof.

The formulation of the present invention comprises, for example: a fast release composition comprising as active ingredients melatonin, piperine and/or capsaicin or a capsaicin analogue (preferably phenylcapsaicin), and vitamin D, preferably vitamin D3, vitamin B6, and optionally Zea *mays* extract and/or capsaicin or a capsaicin analogue (preferably phenylcapsaicin); a medium release composition comprising an extract of *Valeriana officinalis* and/or an extract of *Melissa officinalis;* and a prolonged release composition comprising as active ingredient an extract of *Griffonia* (or *Griffonia simplicifolia*) or 5-hydroxytryptophan (5-HTP) and *Passiflora incarnata* extract and extract of *Eschscholzia californica,* or Zea *mays* extract, or Zea *mays* extract and extract of *Passiflora incarnata* and extract of *Eschscholzia californica,* or gamma-aminobutyric acid (GABA) or a salt or an ester thereof.

According to an embodiment of the formulation of the present invention (comprising the fast-, medium- and prolonged- release compositions), the fast release composition further comprises a vitamin B6, and optionally Zea *mays* extract and/or capsaicin or a capsaicin analogue (preferably phenylcapsaicin); preferably, the fast release composition comprises as active ingredient melatonin, piperine, vitamin D (or D3), vitamin B6 and, optionally, Zea *mays* extract and/or capsaicin or a capsaicin analogue (preferably phenylcapsaicin).

The formulation of the present invention, for example, comprises: a fast release composition comprising as active ingredient melatonin, piperine and/or capsaicin or a capsaicin analogue (preferably phenylcapsaicin), vitamin D (or D3) and vitamin B6; a medium release composition comprising an extract of *Valeriana officinalis* and/or an extract of *Melissa officinalis;* a prolonged release composition comprising as active ingredient an extract of *Griffonia* (or *Griffonia simplicifolia*) or 5-hydroxytryptophan (5-HTP).

According to a preferred embodiment of the formulation of the present invention, the formulation comprises or, alternatively, consists of: the fast release composition comprising or, alternatively, consisting of (as active ingredient) melatonin, piperine and/or capsaicin or a capsaicin analogue (preferably phenylcapsaicin), vitamin D (or D3) and vitamin B6, the medium release composition comprising or, alternatively, consisting of (as active ingredient) extract of *Valeriana officinalis* and extract of *Melissa officinalis,* and the prolonged release composition comprising or, alternatively, consisting of (as active ingredient) extract of *Griffonia* (or *Griffonia simplicifolia*) or 5-hydroxytryptophan, and extract of *Passiflora incarnata* and extract of *Eschscholzia californica.*

Melatonin (IUPAC name N-[2-(5-Methoxy-1H-indol-3-yl)ethyl]acetamide, example of CAS nr. 73-31-4) is a hormone primarily released by the pineal gland.

Piperine is the alkaloid (IUPAC name (2E,4E)-5-(2H-1,3-Benzodioxol-5-yl)-1-(piperidin-1-yl)penta-2,4-dien-1-one, example of CAS nr. 94-62-2) comprised in pepper (black pepper and long pepper). Piperine is also known as piperoylpiperidin or bioperin. It is typically extracted from black pepper applying methods and devices known to the skilled person in the art (e.g. extraction with organic solvents; or aqueous extraction using hydrotropic solutions). Piperine is also chemically synthesized. In the contest of the present invention, piperine can be substituted by a capsaicin or a capsaicin analogue (preferably phenylcapsaicin).

*Valeriana officinalis* L. (synonyms: valerian) is a perennial flowering plant in the family *Caprifoliaceae.* Advantageously, the *Valeriana officinalis* extract comprised in the formulation of the present invention comprises valerenic acid in a range from 0.05% to 10% (wt/wt), preferably from 0.1% to 5%, for example a *Valeriana officinalis* dry extract standardized in circa 0.8% wt valeric acid.

*Melissa officinalis* L. (synonyms: lemon balm, balm, common balm, or balm mint) is a perennial herbaceous plant in the mint family *Lamiaceae.* Advantageously, the *Melissa officinalis* extract comprised in the formulation of the present invention comprises rosmarinic acid in a range from 0.5% to 10% (wt/wt), preferably from 1% to 5%, for example a *Melissa officinalis* dry extract standardized in circa 2% wt rosmarinic acid.

*Eschscholzia californica* (synonyms: California poppy, golden poppy, California sunlight or cup of gold) is a species of flowering plant in the family *Papaveraceae.*

*Eschscholzia californica* has been shown to contain the following alkaloids: califomidine, escholtzine, protopine, N-Methyllaurotetanine, caryachine, 0-methylcaryachine, and the pavine alkaloid, 6S,12S-neocaryachine-7-0-methyl ether N-metho salt. The main alkaloids present in the leaves are the isoquinoline alkaloids califomidine, escholtzine and protopine which are associated with its known therapeutic properties. Aqueous/ethanol extract of *Eschscholzia californica* (root and/or leaves) comprises isoquinoline alkaloids. Advantageously, the *Eschscholzia californica* extract comprised in the formulation of the present invention comprises alkaloids in a range comprised from 0.05% to 5% (wt/wt), preferably from 0.1% to 1%, for example an *Eschscholzia californica* dry extract standardized in circa 0.2% wt alkaloids.

*Passiflora incarnata* L. (synonyms: maypop, purple passionflower, true passionflower, wild apricot, and wild passion vine) is a fast-growing perennial vine in the family *Plassifloraceae. Passiflora incarnata* contains flavonoids and alkaloids and leaves containing the greatest concentration of flavonoids. Other flavonoids present in *Passiflora incarnata* include chrysin, apigenin, luteolin, quercetin, kaempferol, and isovitexin. Advantageously, the *Passiflora incarnata* extract comprised in the formulation of the present invention comprises flavonoids in a range from 0.5% to 10% (wt/wt), preferably from 1% to 5%, for example a *Passiflora incarnata* dry extract standardized in circa 2% wt flavonoids.

Griffonia is a genus of flowering plants in the legume family, *Fabaceae. Griffonia* comprises the following species: *Griffonia physocarpa, Griffonia simplicifolia,*
*Griffonia speciosa, Griffonia tessmannii.* The seeds of *Griffonia simplicifolia* (DC.; Baill.) are used as an herbal supplement for their 5-hydroxytryptophan (5-HTP) content. Advantageously, the *Griffonia* or *Griffonia simplicifolia* extract comprised in the formulation of the present invention comprises 5-hydroxytryptophan in a range from 80% to 99,9% (wt/wt), preferably 90% to 99%, for example a *Griffonia* or *Griffonia simplicifolia* dry extract standardized in circa 98% wt 5-hydroxytryptophan.

5-Hydroxytryptophan (5-HTP, also known as oxitriptan) is a naturally occurring amino acid; IUPAC name 2-amino-3-(5-hydroxy-1H-indol-3-yl)propanoic acid and example of CAS nr 4350-09-8.

The botanical extracts of the present invention are obtained applying methods and devices known to the skilled person in the art.

In a preferred example of the controlled-release formulation of the present invention, the formulation comprises or, alternatively, consists of: the fast release composition comprising or, alternatively, consisting of (as active ingredient) melatonin, piperine, vitamin B6 and vitamin D3; the medium release composition comprising or, alternatively, consisting of (as active ingredient) extract of *Valeriana officinalis* standardized in circa 0.1% to 5% wt valerenic acid (for example, circa 0.8% wt) and extract of *Melissa officinalis* standardized in circa 1% to 5% wt rosmarinic acid (for example, circa 2% wt); and the prolonged release composition comprising or, alternatively, consisting of (as active ingredient) extract of *Griffonia* (or *Griffonia simplicifolia*) standardized in circa 80% to 99% wt 5-hydroxytryptophan (for example, circa 98% wt), extract of *Passiflora incarnata* standardized in circa 1% to 5% wt flavonoids (for example, circa 2% wt), and extract of *Eschscholzia californica* standardized in circa 0.1% to 1% wt alkaloids (for example, circa 0.2% wt).

The terms "titled in" "standardizes in", related to the content of a botanical extract, are synonyms and mean "comprising".

According to an example of the controlled-release formulation of the present invention, the formulation comprises or, alternatively, consists of (as active ingredients):
- the fast release composition comprising melatonin in a rate of 0.5 mg to 20 mg (e.g. circa 1 mg, 1.5 mg, 2 mg, 2.5 mg, 5 mg, 10 mg, or 15 mg), piperine in a rate of 0.5 mg to 50 mg (e.g. circa 1 mg, 2 mg, 3 mg, 4 mg, 5 mg, 6 mg, 7 mg, 10 mg, 20 mg, 30 mg, or 40 mg), and vitamin D3 in a rate of 0.5 µg to 50 µg (e.g. circa 2 µg, 3 µg, 4 µg, 5 µg, 6 µg, 7 µg, 8 µg, 9 µg, 10 µg, 20 µg, 30 µg, or 40 µg,) and, optionally, vitamin B6 in a rate of 0.1 mg to 20 mg; preferably melatonin in a rate of 1 mg to 5 mg, piperine in a rate of 2.5 mg to 10 mg, vitamin D3 in a rate of 1 µg to 10 µg, and, optionally, vitamin B6 in a rate of 0.5 mg to 5 mg, and, optionally, Zea *mays* extract in a rate of 50 mg to 300 mg, and/or, optionally, capsaicin or a capsaicin analogue (preferably phenylcapsaicin) in a rate from 0,5 mg to 300 mg, preferably from 10 mg to 200 mg, more preferably from 50 mg to 100 mg.;
- the medium release composition comprising extract of *Valeriana officinalis* in a rate of 10 mg to 500 mg (e.g. circa 50 mg, 100 mg, 150 mg, 200 mg, 250 mg, 300 mg, or 400 mg) and/or extract of *Melissa officinalis* in a rate of 10 mg to 500 mg (e.g. circa 50 mg, 100 mg, 150 mg, 200 mg, 250 mg, 300 mg, or 400 mg); preferably extract of *Valeriana officinalis* in a rate of 50 mg to 200 mg and/or extract of *Melissa officinalis* in a rate of 50 mg to 200 mg; and
- the prolonged release composition comprising:
   (i) extract of *Griffonia* (or *Griffonia simplicifolia*; comprising 5-HTP in a rate of 90%-99% wt) or 5-hydroxytryptophan in a rate of 1 mg to 300 mg (e.g. circa 10 mg, 15 mg, 20 mg, 25 mg, 30 mg, 35 mg, 40 mg, 45mg, 50 mg, 60 mg, 100 mg, 150 mg, or 200 mg) and, optionally, extract of *Passiflora incarnata* in a rate of 10 mg to 500 mg (*e.g.* circa 50 mg, 100 mg, 150 mg, 200 mg, 250 mg, 300 mg, or 400 mg) and/or extract of *Eschscholzia californica* in a rate of 10 mg to 500 mg (e.g. circa 50 mg, 100 mg, 150 mg, 200 mg, 250 mg, 300 mg, or 400 mg); preferably extract of *Griffonia* (or 5-HTP) in a rate of 10 mg to 60 mg and, optionally, extract of *Passiflora incarnata* in a rate of 50 mg to 200 mg and/or extract of *Eschscholzia californica* in a rate of 50 mg to 200 mg; or
   (ii) Zea *mays* extract in a rate of 50 mg to 250 mg, preferably in a rate of 70 mg to 200 mg, and, optionally, extract of *Passiflora incarnata* in a rate of 10 mg to 500 mg and/or extract of *Eschscholzia californica* in a rate of 10 mg to 500 mg, preferably extract of *Passiflora incarnata* in a rate of 50 mg to 200 mg and/or extract of *Eschscholzia californica* in a rate of 50 mg to 200 mg; or
   (iii) gamma-aminobutyric acid (GABA) or a salt or an ester thereof in a rate of 1 mg to 300 mg, preferably in a rate of 50 mg to 200 mg, and, optionally, extract of *Passiflora incarnata* in a rate of 10 mg to 500 mg and/or extract of *Eschscholzia californica* in a rate of 10 mg to 500 mg, preferably extract of *Passiflora incarnata* in a rate of 50 mg to 200 mg and/or extract of *Eschscholzia californica* in a rate of 50 mg to 200 mg. In the context of the present invention the component Gaba in the prolonged release second layer can be replaced or can be substituted by a milk protein hydrolyzed in an amount from 50 mg to 250 mg, preferably from 100 mg to 200 mg, for example 150 mg; said milk protein hydrolyzed can be the product Lactium^{®}.

The product Lactium^{®} is a milk protein hydrolyzed in spray-dried powder that naturally contains a bioactive decapeptide with soothing properties. Lactium^{®} is perfectly adapted to both dietary supplements (tablets, capsules, softgels, gums...) and functional foods (bars, chocolate, beverages...).

Lactium^{®} has been clinically proven to reduce stress-related symptoms: weight troubles, sleep disorders, smoking, mood swings, libido decrease, memory and concentration impairment, digestive disorders.

The milk protein hydrolyzed which find applicability in the present invention may have:
- A total moisture from 3% to 8%, preferably 4.5%;
- A total protein from 16% to 90%, preferably 80%;
- A total fat from 0.1% to 1%, preferably 0.5%;
- A total carbohydrate from 0.05% to 0.5%, preferably 0.3%.

For example, said milk protein hydrolyzed may have the following aminogram (per 100 g protein)
- Alanine 2.85 %
- Arginine 3.50 %
- Aspartic acid +Asparagine 6.69 %
- Glutamic acid + Glutamine 21.62 %
- Glycine 1.99 %
- Histidine (EAA) 2.74 %
- Isoleucine (BCAA-EAA) 4.92 %
- Leucine (BCAA-EAA) 8.81 %
- Lysine (EAA) 7.22 %
- Methionine (EAA) 2.35 %
- Phenylalanine (EAA) 4.97 %
- Proline 11 %
- Serine 5.34 %
- Threonine (EAA) 3.40 %
- Tryptophan (EAA) 1.24 %
- Valine (BCAA-EAA) 5.78 %

The present quantities of active ingredients in the formulation of the present invention must be intended as quantity for single dosage and/or quantities for daily dosage. Preferably the daily dosage of the formulation of the present invention is administered to a subject in need in a single dose, for example before (15-30 minutes to 60 minutes) going to sleep.

In a preferred example of the controlled-release formulation of the present invention, the formulation comprises or, alternatively, consists of: the fast release composition comprising melatonin in a rate of 0.5 mg to 20 mg, piperine in a rate of 0.5 mg to 50 mg, vitamin D3 in a rate of 0.5 µg to 50 µg, and, optionally, vitamin B6 in a rate of 0.1 mg to 20 mg; preferably melatonin in a rate of 1 mg to 5 mg, piperine in a rate of 2.5 mg to 10 mg, vitamin D3 in a rate of 1 µg to 10 µg, and, optionally, vitamin B6 in a rate of 0.5 mg to 5 mg; the medium release composition comprising extract of *Valeriana officinalis* in a rate of 10 mg to 500 mg and extract of *Melissa officinalis* in a rate of 10 mg to 500 mg, preferably extract of *Valeriana officinalis* in a rate of 50 mg to 200 mg and/or extract of *Melissa officinalis* in a rate of 50 mg to 200 mg; and the prolonged release composition comprising extract of *Griffonia* (or *Griffonia simplicifolia*; comprising 5-HTP in a rate of 90%-99% wt) or 5-HTP in a rate of 1 mg to 300 mg, extract of *Passiflora incarnata* in a rate of 10 mg to 500 mg and extract of *Eschscholzia californica* in a rate of 10 mg to 500 mg, preferably extract of *Griffonia* or 5-HTP in a rate of 10 mg to 60 mg, extract of *Passiflora incarnata* in a rate of 50 mg to 200 mg and extract of *Eschscholzia californica* in a rate of 50 mg to 200 mg.

In a more preferred example of the formulation of the present invention, the formulation comprises or, alternatively, consists of: the fast release composition comprising melatonin in a rate of 1 mg to 5 mg (e.g. circa 1.5-2 mg), piperine in a rate of 2.5 mg to 10 mg (e.g. circa 4-6 mg), vitamin D3 in a rate of 1 µg to 10 µg (*e.g.* circa 4-6 mg) and vitamin B6 in a rate of 0.5 mg to 5 mg (e.g. circa 1-2 mg); the medium release composition comprising extract of *Valeriana officinalis* in a rate of 50 mg to 200 mg (e.g. circa 95-105 mg) and/or extract of *Melissa officinalis* in a rate of 50 mg to 200 mg (e.g. circa 95-105 mg); and the prolonged release composition comprising extract of *Griffonia* (or *Griffonia simplicifolia*; comprising 5-HTP in a rate of 90%-99% wt) or 5-HTP in a rate of 10 mg to 60 mg (e.g. circa 25-35 mg), extract of *Passiflora incarnata* in a rate of 50 mg to 200 mg (e.g. circa 95-105 mg) and extract of *Eschscholzia californica* in a rate of 50 mg to 200 mg (e.g. circa 95-105 mg).

The compositions of the present invention or the single compositions comprised in the controlled-release formulation of the present invention (fast-, medium- and/or prolonged- release compositions) preferably comprise at least one pharmaceutical or food grade additive and/or excipient, i.e. a substance devoid of therapeutic activity suitable for pharmaceutical or food use. In the context of the present invention the acceptable ingredients for pharmaceutical or food use comprise all auxiliary substances known to the man skilled in the art such as, by way of non-limiting examples, diluents, solvents, solubilizers, thickeners, sweeteners, flavour enhancers, colourants, lubricants, surfactants, antimicrobials, antioxidants, preservatives, pH stabilizing buffers and mixtures thereof. Non-limiting examples of such substances are phosphate buffers (dicalcium phosphate), magnesium stearate, silicone dioxide, cross-linked sodium carboxymethylcellulose (sodium croscarmellose), microcrystalline cellulose, hydroxypropyl methylcellulose (Hypromellose), natural or artificial flavours.

Hypromellose (INN), shortening for hydroxypropyl methylcellulose (HPMC, example of CAS nr 9004-65-3), is a semisynthetic, inert, viscoelastic polymer used as an excipient and controlled-delivery component in oral medicaments. The Codex Alimentarius code (E number, according to the European legislation) of Hypromellose is E464.

In the controlled-release formulation of the present invention, the medium release composition may comprise, for single dosage and/or for daily dose, Hypromellose in a rate of 10 mg to 100 mg (e.g. 20 mg, 30 mg, 40 mg, 50 mg, 60 mg, 70mg, 80 mg, or 90 mg), preferably in a rate of 30 mg to 90 mg, for example circa 50-60 mg.

In the controlled-release formulation of the present invention, the prolonged release composition may comprise, for single dosage and/or for daily dose, Hypromellose in a rate of 100 mg to 200 mg (e.g. 120 mg, 130 mg, 140 mg, 150 mg, 160 mg, 170mg, 180 mg, or 190 mg), preferably in a rate of 75 mg to 135 mg, for example circa 100-110 mg.

Advantageously, the controlled-release formulations or compositions of the present invention (according to any one of the disclosed embodiments and examples) may further comprise as active ingredient at least one compound or botanic extract selected in the group comprising or, alternatively, consisting of: a vitamin of group B other than vitamin B6, organic and/or inorganic salts of magnesium, selenium, zinc, hawthorn, chamomile, hops, *scutellaria,* antioxidants, L-tryptophan, corn or maize derived from Zea *mays* and mixtures thereof.

The compositions and controlled-release formulations of the present invention according to any one of the disclosed embodiments or examples are suitable as pharmaceutical formulations, formulations for medical device, formulations for dietary supplement or food for special medical purpose.

The compositions and controlled-release formulations for oral administration according to the present invention may be in solid form, such as tablet (made of single and individual layers, for example one layer, two individual layers, three individual layers or four individual layers), chewable tablet, capsule, lozenge, granules or powder; it is preferably in solid form, more preferably in form of tablet (multi layers tablet).

It is an object of the present invention to provide the mixtures, compositions and/or controlled-release formulations according to the present invention for use as a medicament.

It is an object of the present invention to provide the mixtures, compositions and/or controlled-release formulations according to the present invention for use in a method of preventive and/or curative treatment of sleep disorders and associated symptoms thereof in a subject in need thereof.

Sleep disorders and/or associated symptoms thereof treated by the formulation of the present invention (according to any one of the disclosed embodiments and examples) are, preferably, selected from the group comprising or, alternatively, consisting of: insomnia, inability to fall asleep, difficulty falling asleep, early awakening, and interrupted sleep, excessive daytime sleepiness, symptoms of shift work disorder, jet lag disorder (JLD) and symptoms thereof, dream disorders, dream disorders associated to Post-Traumatic Stress syndrome.

It is an object of the present invention to provide a method of treatment, preventive and/or curative, of prostate pathologies and/or symptoms, of sleep disorders and associated symptoms thereof, as described above, in a subject in need by administering to the subject a therapeutically effective amount of the mixtures or compositions or controlled-release formulations of the present invention.

It is an object of the present invention to provide a non-medical (non-therapeutic) use of conditions linked to sleep related problems in a healthy subject, by administering to said subject an adequate amount of the mixtures or compositions or controlled-release formulations of the present invention.

The mixtures or compositions or controlled-release formulations of the present invention are for use in a method of treatment of sleep disorders and/or symptoms associated thereof (as mentioned above) both when administered to a subject as a sole therapy, and when administered as an adjuvant to at least one other therapy or composition for aiding sleep.

According to the embodiments reported in the present description, the mixtures or compositions or controlled-release formulations of the present invention allow an effective therapeutic or non-therapeutic treatment of diseases, symptoms, disorders and/or disfunctions associated with sleep cycle dysfunctions. Particularly, the mixtures or compositions or controlled-release formulations of the present invention ensure a relaxation of the treated subject in the short term which leads the subject to fall asleep rapidly and, furthermore, they guarantee the duration of deep sleep protecting the subject against nocturnal awakenings or early interruption of sleep. The disclosed mixtures or compositions or controlled-release formulations do not have any relevant adverse effects.

The term "subject/s" in the context of the present invention indicates mammals (animals and humans).

Unless otherwise specified, the term "the formulation/composition comprises a component in the range of x to y" means that said component can be present in the composition in all the quantities defined by said range, extremes of the range included.

The term "therapeutically effective amount" refers to the amount of active compound/s eliciting the biological or medicinal response in a tissue, system, mammal, or human being that is sought and defined by an individual, researcher, veterinarian, physician, or other clinician or health worker.

In the context of the present invention, the term "medical device" is used according to the Medical Devices Regulation (UE) 2017/745 (MDR).

### EXAMPLE of active ingredients

Hereinafter, the active ingredients comprised (essential or not-essential) in the controlled release formulation according to the present invention will be described in detail with reference to the examples.

Melatonin (commercial product by C.F.M., Italy): assay HPLC 99.0% min, total impurities HPLC 1% MAX, melting range 117°C-119°C, sulphated ash 0.1% MAX, loss on drying 0.2% MAX.

Piperine (commercial product by Sochim International spa, Italy) commercial name BIOPERINE^{®} *Piper nigrum* PE 95%; a standardized powder extract obtained by patented process from the fruit of black pepper plants (botanical name: *Piper nigrum* L., botanical family: *Piperaceae*, part of plant used: fruits); piperine content (by HPLC method): from 15% to 99%, preferably from 20% to 60%, for example 30%, or 40% or 50%, not less than 95.0% and not more than 102.0% on dried basis; Physical characteristic: appearance: off white to greenish yellow powder; loss on drying: not more than 2.0%; solubility: soluble in alcohol, insoluble in water; ash content: sieve test (passes through):-80 Mesh not more than 0.1%, not less than 95.0%; bulk density (tapped): between 0.55 g/ml - 0.85 g/ml; bulk density (loose): between 0.30 g/ml - 0.65 g/ml; identification: by HPLC; final extract ratio: 50:1; Grade: food grade.

Vitamin D3 (commercial product by BASF) commercial name Dry Vitamin D3 100 GFP: Vitamin D3 (incl. pre-D) (HPLC) ≥ 100000 IU/g (test method BCPL325A; Loss on drying (gravimetric) (105°C) ≤ 5 % (test method BCPL0048); Through mesh 20 USP (sieving) 100% (test method BCPL049A); Through mesh 40 USP (sieving) ≥ 90 % (test method BCPL049C); Through mesh 100 USP (sieving) ≤ 15 % (test method BCPL049H).

Vitamin B6 (commercial product by Nutrifoods) commercial name VITAMIN B6 HCI (Pyridoxine HCI): molecular formula C8H12CINO3; molecular weight 205.60 g/mol; CAS Nr 58-56-0; origin synthetic; assay (anhydrous basis; method Ph. Eur. / EP) 99.0 - 101.0 % (98.0 - 102.0 % method USP); loss on dry ≤0.5 %; sulphated ash ≤0.1 %; pH 2.4-3.0; residual solvent ≤0.5 %; food grade.

Griffonia extract (commercial product by Vivatis Pharma, Italy) commercial name Griffonia seed extract from 15% to 99%, preferably from 15% to 50%, more preferably from 20% to 40%, for example 25%, or 30%, or 35%, or 45%, or 60%, or 70%, or 98% L-5-Hydroxytryptophan: botanical source *Griffonia simplicifolia,* plant part seeds, solvents used water & ethanol (country of origin China); assay: L-5-hydroxytryptophan ≥ 98% (method HPLC); appearance fine powder; identification by HPTLC; sieve analysis 90% through 80 mesh (method USP32 <786>); total ash ≤ 1.0% (method Eur. Ph. 6.0 <2.8.16>); Specific rotation -34.7° to -30.9° (method Eur. Ph. 6.0 <2.9.13>).

Valeriana (commercial product by Tradichem) commercial name VALERIAN DRY EXTRACT 0.8%: botanical name *Valerian Officinalis,* plant part used root; appearance powder; chemical and physical properties: Mesh size 100% through 80 meshes; loss on drying < 5 %; solubility moderately soluble; bulk density 0.3 g/ml - 0.85 g/ml; residual solvent (ethanol) < 0.5%; valerenic acid (HPLC) 0.8% -1.0%.

Melissa (commercial product by MB Med.) commercial name E.S. MELISSA 2%A.: botanical name: *Melissa officinalis* L., botanical part: leaf, origin: Europe, carrier: maltodextrin, excipient: silica (E551), extraction solvent: ethanol; appearance: powder; loss on drying (method EP 9th) % =< 8.0; total ash (method EP 9th) % =< 10.0; apparent density (method M.B.) g/l 450-650; particle size not less pass 300 um (50 Mesh) % => 90; Rosmarinic acid (method HPLC) % => 2.0.

Passiflora (commercial product by Plantex) commercial name PASSION FLOWER DRY EXTRACT: botanical kind *Passifloraceae Passiflora incarnata* L., part of the plant aerial part, extraction solvent ethanol, water (50 : 50 V/V), description beige to brown powder; carrier wheat maltodextrin; bulk density 0.30 to 0.70 (Eur.Ph. VIII°ed 2.9.34; loss on drying < 8.00% (Eur.Ph. VIII°ed 2.8.17); total ash < 13.00% (Eur. Ph. VIII° Ed 2.4.16); screening min. 95% < 500 µm (internal method).

*Eschscholzia* (commercial product by MB Med.) commercial name E.S. ESCOLZIA MIN. 0,2%P. (CALIFORNIAN POPPY EXT): botanical name: *Eschscholtzia calif.* Cham., botanical part: herb, origin: Europe, carrier: maltodextrin, excipient: silica (E551), extraction solvent: ethanol; appearance: powder; loss on drying (method EP 9th) % =< 7.0; total ash (method EP 9th) % =< 15.0; apparent density (method M.B.) 450 g/l -650 g/l; particle size not less pass 300 um (50 Mesh) % => 90; total alkaloids (included californidine) expressed as protopine (method HPLC) % => 2.0.

### EXAMPLE of controlled-release formulations

Hereinafter, the present invention will be described in detail with reference to examples. However, the embodiments and examples of the present invention may be modified into various other forms, and the scope of the present invention should not be interpreted as being restricted to the embodiments and examples.

In the following Examples 1 to 5, the layers may be stacked to form the tablet in the order III-II-I (*i.e.* prolonged release layer between the medium and the fast release layers) or in any other possible order, for example in the order II-III-I (i.e. medium release layer between the prolonged and the fast release layers). The order III-II-I (prolonged release layer between the medium and the fast release layers) is the preferred one.

Example 1: a three layers tablet for oral administration having the formula disclosed in Table 1 (daily dose).

**Table 1**

| | Ingredient | dose in mg |
|---|---|---|
| Medium release layer III | *Valeriana* dry extract std. 0.8% valerenic acid | 50-150 |
| | *Melissa* dry extract std. 2% rosmarinic acid | 50-150 |
| | additives/excipients | Q.S. |
| Prolonged Release layer II | *Griffonia* dry extract std 98% 5-HTP | 10-60 |
| | additives/excipients | Q.S. |
| Fast release layer I | Melatonin | 1-5 |
| | Piperine (95% wt) | 2.5-10 |
| | Vitamin D3 | 1-10 mcg |
| | additives/excipients | Q.S. |
| | TOTAL | 1000 |

Example 2: a three layers tablet for oral administration having the formula disclosed in Table 2 (daily dose).

**Table 2**

| | Ingredient | dose in mg |
|---|---|---|
| Medium release layer III | *Valeriana* dry extract std. 0.8% valerenic acid | 50-150 |
| | *Melissa* dry extract std. 2% rosmarinic acid | 50-150 |
| | Hypromellose | 75-135 |
| | additives/excipients | Q.S. |
| Prolonged Release layer II | *Griffonia* dry extract std 98% 5-HTP | 10-60 |
| | Hypromellose | 30-90 |
| | additives/excipients | Q.S. |
| Fast release layer I | Melatonin | 1-5 |
| | Piperine (95% wt) | 2.5-10 |
| | Vitamin D3 | 1-10 mcg |
| | additives/excipients | Q.S. |
| | TOTAL | 1000 |

Example 3: a three layers tablet for oral administration having the formula disclosed in Table 3 (daily dose).

**Table 3**

| | Ingredient | dose in mg |
|---|---|---|
| Medium release layer III | *Valeriana* dry extract std. 0.8% valerenic acid | 50-150 |
| | *Melissa* dry extract std. 2% rosmarinic acid | 50-150 |
| | Hypromellose | 75-135 |
| | additives/excipients | Q.S. |
| Prolonged Release layer II | *Griffonia* dry extract std 98% 5-HTP | 10-60 |
| | *Passiflora* dry extract std. 2% flavonoids | 50-150 |
| | *Eschscholzia* dry extract std. 0.2% alkaloids | 50-150 |
| | Hypromellose | 30-90 |
| | additives/excipients | Q.S. |
| Fast release layer I | Melatonin | 1-5 |
| | Piperine (95% wt) | 2.5-10 |
| | Vitamin D3 | 1-10 mcg |
| | additives/excipients | Q.S. |
| | TOTAL | 1000 |

Example 4: a three layers tablet for oral administration having the formula disclosed in Table 4 (daily dose).

**Table 4**

| | Ingredient | dose in mg |
|---|---|---|
| Medium release layer III | *Valeriana* dry extract std. 0.8% valerenic acid | 50-150 |
| | *Melissa* dry extract std. 2% rosmarinic acid | 50-150 |
| | Microcrystalline cellulose | Q.S. |
| | Hypromellose | 75-135 |
| | Dicalcium phosphate | Q.S. |
| | Silicon dioxide | Q.S. |
| | Stearate magnesium | Q.S. |
| | Dye | Q.S. |
| Prolonged Release layer II | *Griffonia* dry extract std 98% 5-HTP | 10-60 |
| | *Passiflora* dry extract std. 2% flavonoids | 50-150 |
| | *Eschscholzia* dry extract std. 0.2% alkaloids | 50-150 |
| | Dicalcium phosphate | Q.S. |
| | Silicon dioxide | Q.S. |
| | Stearate magnesium | Q.S. |
| | Hypromellose | 30-90 |
| Fast release layer I | Melatonin | 1-5 |
| | Piperine (95% wt) | 2.5-10 |
| | Vitamin D3 | 1-10 mcg |
| | Microcrystalline cellulose | Q.S. |
| | Dicalcium phosphate | Q.S. |
| | Croscarmellose | Q.S. |
| | Silicon dioxide | Q.S. |
| | Stearate magnesium | Q.S. |
| | Dye | Q.S. |
| | TOTAL | 1000 |

Example 5: a three layers tablet for oral administration having the formula disclosed in Table 5 (daily dose).

**Table 5**

| | Ingredient | dose in mg |
|---|---|---|
| Medium release layer III | *Valeriana* dry extract std. 0.8% valerenic acid | 50-150 |
| | *Melissa* dry extract std. 2% rosmarinic acid | 50-150 |
| | Microcrystalline cellulose | Q.S. |
| | Hypromellose | 75-135 |
| | Dicalcium phosphate | Q.S. |
| | Silicon dioxide | Q.S. |
| | Stearate magnesium | Q.S. |
| | Dye | Q.S. |
| Prolonged Release layer II | *Griffonia* dry extract std 98% 5-HTP | 10-60 |
| | *Passiflora* dry extract std. 2% flavonoids | 50-150 |
| | *Eschscholzia* dry extract std. 0.2% alkaloids | 50-150 |
| | Dicalcium phosphate | Q.S. |
| | Silicon dioxide | Q.S. |
| | Stearate magnesium | Q.S. |
| | Hypromellose | 30-90 |
| Fast release layer I | Melatonin | 1-5 |
| | Piperine (95% wt) | 2.5-10 |
| | Vitamin B6 | 0.5-5 |
| | Vitamin D3 | 1-10 mcg |
| | Microcrystalline cellulose | Q.S. |
| | Dicalcium phosphate | Q.S. |
| | Croscarmellose | Q.S. |
| | Silicon dioxide | Q.S. |
| | Stearate magnesium | Q.S. |
| | Dye | Q.S. |
| | TOTAL | 1000 |

Example 6: a three layers tablet for oral administration having the formula disclosed in Table 5.A (daily dose)

**Table 5.A**

| | Ingredient | dose in mg |
|---|---|---|
| Medium release layer III | *Valeriana* dry extract std. 0.8% valerenic acid | 100 |
| | *Melissa* dry extract std. 2% rosmarinic acid | 100 |
| Prolonged Release layer II | *Griffonia* dry extract std 98% 5-HTP | 30 |
| | *Passiflora* dry extract std. 2% flavonoids | 100 |
| | *Eschscholzia* dry extract std. 0.2% alkaloids | 100 |
| Fast release layer I | Melatonin | 1-2 |
| | Piperine (95% wt) | 5 |
| | Vitamin B6 | 1.4* |
| | Vitamin D3 | 5 mcg* |

| | | |
|---|---|---|
| * (100% NRV; Nutritient Reference Value) | | |

Example 7: a three layers tablet for oral administration having the formula disclosed in Table 5.B (daily dose)

**Table 5.B**

| | Ingredient | dose in mg |
|---|---|---|
| Medium release layer III | *Valeriana* dry extract std. 0.8% valerenic acid | 100 |
| | *Melissa* dry extract std. 2% rosmarinic acid | 100 |
| Prolonged Release layer II | Zea *mays* (corn extract) | 250 |
| Fast release layer I | Melatonin | 1-2 |
| | Piperine (95% wt) | 5 |
| | Vitamin B6 | 1.4* |
| | Vitamin D3 | 5 mcg* |

| | | |
|---|---|---|
| * (100% NRV; Nutritient Reference Value) | | |

Example 8: a three layers tablet for oral administration having the formula disclosed in Table 5.C (daily dose)

**Table 5.C**

| | Ingredient | dose in mg |
|---|---|---|
| Medium release layer III | *Valeriana* dry extract std. 0.8% valerenic acid | 100 |
| | *Melissa* dry extract std. 2% rosmarinic acid | 100 |
| Prolonged Release layer II | Zea *mays* (corn extract) | 100 |
| | *Passiflora* dry extract std. 2% flavonoids | 50 |
| | *Eschscholzia* dry extract std. 0.2% alkaloids | 50 |
| Fast release layer I | Melatonin | 1-2 |
| | Zea *mays* (corn extract) | 150 |
| | Piperine (95% wt) | 5 |
| | Vitamin B6 | 1.4* |
| | Vitamin D3 | 5 mcg* |

| | | |
|---|---|---|
| * (100% NRV; Nutritient Reference Value) | | |

Example 9: a three layers tablet for oral administration having the formula disclosed in Table 5.D (daily dose)

**Table 5.D**

| | Ingredient | dose in mg |
|---|---|---|
| Medium release layer III | *Valeriana* dry extract std. 0.8% valerenic acid | 100 |
| | *Melissa* dry extract std. 2% rosmarinic acid | 100 |
| Prolonged Release layer II | GABA | 100 |
| | *Passiflora* dry extract std. 2% flavonoids | 80 |
| | *Eschscholzia* dry extract std. 0.2% alkaloids | 50 |
| Fast release layer I | Melatonin | 1-2 |
| | Piperine (95% wt) | 5 |
| | Vitamin B6 | 1.4* |
| | Vitamin D3 | 5 mcg* |

| | | |
|---|---|---|
| * (100% NRV; Nutritient Reference Value) | | |

Example 10: a three layers tablet for oral administration having the formula disclosed in Table 5.E (daily dose)

**Table 5.E**

| | Ingredient | Dose in mg |
|---|---|---|
| Fast release layer I | Melatonin | 1 - 1.95 |
| | Vitamin D | 5 mcg* |
| | Piperine (95% wt) | 5 |
| | Vitamin B6 | 1.4* |
| Prolonged Release layer II | Milk Protein hydrolysate | 150 |
| | Passiflora extract | 30 |
| | Californian Poppy extract | 50 |
| Medium release layer III | Valerian extract | 100 |
| | Lemon Balm extract | 100 |

| | | |
|---|---|---|
| * (100% NRV; Nutritient Reference Value) | | |

EXAMPLE of *in vitro* measure of intestinal and hepatic pass.

### 1. SCOPE

### Phase 1: Assessment of intestinal absorption rate.

For this purpose, intestinal barrier model (transwell, patented model from Novara University, Italy) is used to determine the rate of passage and therefore to estimate a better pass-effect of melatonin in combination with piperine and vitamin D than melatonin alone. In this phase, vitality will also be assessed in the presence of the treatments alone and in combination with melatonin, in addition to melatonin alone.

### Phase 2: Analysis of hepatic absorption.

For this purpose, a co-culture model between intestinal cells and hepatic cells is used to determine a better effect of the combination "melatonin + piperine + vitamin D3" compared to melatonin alone. In this phase the vitality in the presence of the combined treatments will also be assessed compared to the single treatments.

### Phase 3: Study of hepatic intracellular mechanisms.

The study will be conducted on hepatic cells (human HepG2) and involves an analysis of cell viability with a dosage of the melatonin metabolite, as well as an analysis of its receptors and the 2 CYPs mainly involved in the effects of melatonin (1A2 and 3A4).

### 2. MATERIAL and METHOD

- Melatonin (brief, MEL) 1mM
- Piperine (brief, pepper or piper) 5µM
- Vitamin D3 1nM
- Vitamin B6 (brief, vitD) 500µM
- Intestinal cells (Caco-2)
- Hepatic cells (human HepG2)

### PHASE 3: Study of hepatic intracellular mechanisms

In humans, melatonin is metabolised primarily to 6-hydroxymelatonin (6-HMEL), which is further conjugated with sulphate and excreted in the urine. O-demethylation represents a minor reaction. CYP1A1, CYP1A2, and CYP1B1 are all 6-hydroxylated MELs, with CYP2C19 playing a minor role. Among these, CYP1A2 is probably the main hepatic P450 in the 6-hydroxylation of melatonin.

Conversely, the greatest inhibition of melatonin metabolism was observed with the involvement of the CYP2E1 and CYP3A4 family, at the microsomal level. Particularly, the CYP3A4 family is mainly responsible for the O-demethylation of melatonin and to a lesser extent, the 6-hydroxylation of melatonin. Therefore: CYP1A2 is important in the hydroxylation of melatonin, while CYP3A4 provides added value at the level of mitochondrial metabolism (mediator of biotransformation with an inhibitory effect).

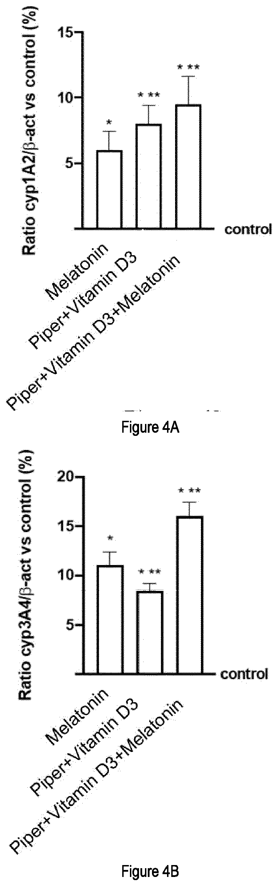

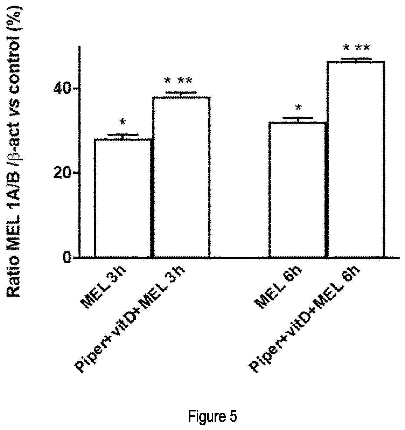

Method (Figures 4 and 5): at the end of each stimulation, cells were lysed in ice with Complete Tablet Buffer (Roche, Basle, Switzerland) supplemented with 2 mM sodium orthovanadate (Na₃VO₄), 1 mM phenylmethanesulfonyl fluoride (PMSF) (Sigma-Aldrich), 1:50 mix Phosphatase Inhibitor Cocktail (Sigma-Aldrich), and 1:200 mix Protease Inhibitor Cocktail (Sigma-Aldrich). According to the standard protocol, 35 µg of protein of each sample was resolved on SDS-PAGE gels, and polyvinylidene difluoride membranes (PVDF, GE, Healthcare Europe GmbH) were incubated overnight at 4°C with the following specific primary antibodies: CYP1A2 (1:1000) and CYP3A4 (1:1000). Protein expression was normalized and verified through anti-β-actin detection (1:1000, Sigma-Aldrich). The results were expressed as means ± SD (% vs. control).

β-act = b-actin protein. Beta-actin, also known as a "housekeeping" protein, is usually used as a loading control, for among others, the integrity of cells, protein degradation, in PCR and Western blotting. Loading controls are essential for proper interpretation of western blots. They can be used to normalize the levels of protein detected by confirming that protein loading is the same across the gel. Beta-actin is an isoform of actin proteins. Actin proteins are highly conserved proteins that are involved in cell motility, structure, and integrity. There are six different but highly conserved actin isoforms in vertebrates. Four of these isoforms are primarily expressed in striated and smooth muscle cells, whereas beta-actin and gamma-actin isoforms are ubiquitously expressed.

Control= A control group can be the set of cells grown in the same conditions with the stimulated groups but without the agent stimulations. During all the time period, these cells were maintained under exactly the same conditions as the experimental group.

### 3. RESULTS

### 3.1. PHASE 1 RESULTS

### 3.1.a. Intestinal cells - Cell viability

The results are shown in Table 6. A time-dependent trend can be observed for all tested substances. In addition, substances other than melatonin have an efficacy that resolves within 2-4 hours of treatment.

**Table 6**

| Time (h) | Melatonin | | Vitamin D3 | | Piperine | | Vitamin B6 | |
|---|---|---|---|---|---|---|---|---|
| | Average | SD | Average | SD | Average | SD | Average | SD |
| 1 | 13.375 | 0.88388 | 6.91 | 2.70115 | 6.37 | 1.93747 | 11.985 | 0.02121 |
| 2 | 10.905 | 0.13435 | 14.185 | 1.15258 | 30.25 | 1.06066 | 5.215 | 0.30406 |
| 3 | 7.57 | 0.60811 | 18.32 | 0.96167 | 21.625 | 1.94454 | 5.965 | 0.09192 |
| 4 | 6.965 | 0.0495 | 16.325 | 0.95459 | 14.125 | 1.23744 | 9.105 | 2.55266 |
| 5 | 2.57 | 0.60811 | 9.315 | 0.96874 | 12.32 | 0.45255 | 3.565 | 0.61518 |
| 6 | 1.005 | 0.00707 | 0.78 | 0.31113 | 6.74 | 1.04652 | 4.8 | 0.28284 |

### 3.1.b. Intestinal absorption: to determine speed based on the fluorescent tracer, the percentage velocity calculated with respect to the control.

The single substances results are shown in Table 7: the examined substances have a different speed but have a similar trend (peak between 2 hours and 3 hours).

**Table 7**

| X | Group A | Group C | Group D | Group G |
|---|---|---|---|---|
| Speed | Melatonin | Vitamin D3 | Piperine | Vitamin B6 |
| X | Y | Y | Y | Y |
| 30 | 5.00 | 8.00 | 6.00 | 3.00 |
| 1 | 12.00 | 10.00 | 7.00 | 3.00 |
| 2 | 29.00 | 15.00 | 18.00 | 19.00 |
| 3 | 28.00 | 19.00 | 18.00 | 13.00 |
| 4 | 24.00 | 17.00 | 16.00 | 10.00 |
| 5 | 10.00 | 13.00 | 16.00 | 8.00 |
| 6 | 4.00 | 10.00 | 5.00 | 6.00 |

The combination results are shown in Table 8: compared to melatonin alone, the selected combination (melatonin, piperine and vitamin D3) has a higher speed with a peak always between 2 hours and 3 hours of treatment. The data of the combination "Piperine + Vitamin D3" (without melatonin) shows a lower speed value in comparison to the combination "Piperine + Vitamin D3 + melatonin".

**Table 8**

| time (h) | Melatonin | Piper+Vit D | Piper+Vit D + Melatonin |
|---|---|---|---|
| X | Y | Y | Y |
| 1 | 12.00 | 22.88 | 31.00 |
| 2 | 29.00 | 25.91 | 31.41 |
| 3 | 28.00 | 22.59 | 33.00 |
| 4 | 24.00 | 22.33 | 31.20 |

### 3.2. PHASE 2 RESULTS

### 3.2.a. Co-culture model between intestinal cells and hepatic cells

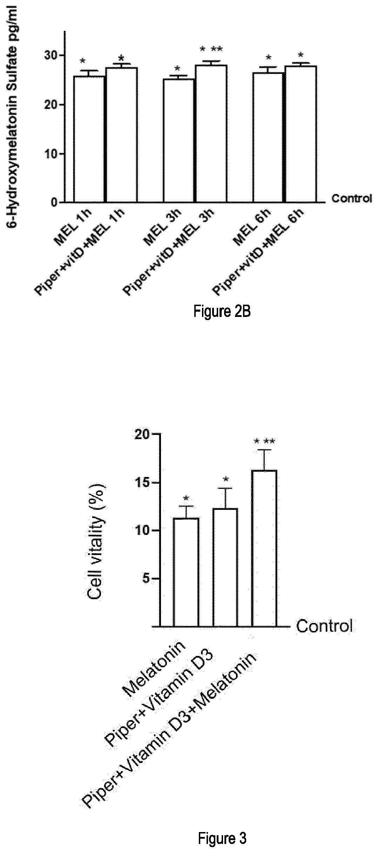

The combination results are shown in Figures 2A (plasmatic pass) and 2B (hepatic pass): the concentration of metabolite (6-hydroxymelatonin sulphate) present in the liver after plasma passage shows that the combination "Piperine + vitamin D3 + melatonin" has a greater absorption than melatonin alone over time (the combination amplifies the effect in 3 hours).

### 3.3: PHASE 3 RESULTS

### 3.3.a. Monolayer hepatic cells: vitality

The results are shown in Figure 3: the combination (melatonin, piperine and vitamin D3) is more effective on vitality (understood as the mitochondrial well-being of the cells) than melatonin alone.

### 3.3.b. Monolayer hepatic cells: CYP 1A2 (inhibitory) and 3A4 (activator)

The results are shown in Figures 4A and 4B: the combination (melatonin, piperine and vitamin D3) increases the activity of CYP 3A4 (activator), suggesting greater bioavailability of the combination versus melatonin alone.

### 3.3.c. Melatonin receptor

Melatonin receptors mediate improvements of liver function and beneficial effects of the pineal hormone seem to be dependent on melatonin receptor activation.

As reported in Figure 5, the activation of melatonin receptors was amplified by the combination (melatonin, piperine and vitamin D3) compared to melatonin alone.

### 6. CONCLUSION

Melatonin undergoes first-pass hepatic processing.

It is possible to modulate melatonin plasma bioavailability with the presence of vitamin D3 and piperine, managing to maximize absorption at circa 3 hours.

### Experimental part II

"Randomized, placebo-controlled clinical trial on the ability of a composition according to the invention to improve sleep quality in subjects with insomnia problems."

Compositions according to the invention as reported in Examples 1-9 were tested (Table 1-5D).

### Main Objective

The main objective of the study is to evaluate the ability of the composition according to the invention, in an objective way, to improve the quality and quantity of sleep (latency time, total sleep time, number of nocturnal awakenings and sleep/wakefulness distribution) after clinical evaluation, with actigraphy and validated scales for patients with insomnia.

### Secondary Objectives

- Moreover, the study is aimed to evaluate the ability of the food supplement to improve the subjective perception of sleep by means of:
- Sleep test (TS: modified from the "Oviedo Sleep Questionnaire"). (www.dormirbien.info/evalua-tu-sueno )
- Health Status and Quality of Life Questionnaire (SF-12)
- Sleep Quality Scale (Pittsburgh or PSQI)
- Insomnia Severity Scale (ISI)
- Electronic Sleep Diary (ESD): www.dormirbien.info/diario
- To assess the tolerability and safety of the supplement under study.
- To know the opinion and degree of satisfaction of the patient and the physician.

### Type of the Study

Clinical trial with the composition according to the invention, without additional drug or medical device.

### Study's design

Single-center, double-blind, randomized, placebo-controlled clinical trial on the ability of a composition according to the invention to improve sleep quality in patients with insomnia, with a 15-day follow-up.

### Randomization process

Assignment to the study group (study supplement) or control group (placebo) is by randomization, from an automatically generated randomization list, in a 1:1 ratio (25 subjects in group A and 25 subjects in group B).

The investigators, after obtaining the signature of the informed consent, assign the patient code, consecutively with correlative numbers, and record it on the patient identification sheet in the patient's file and in the patient's clinical history.

During the first week, sleep is recorded by means of the actigraphy without the patient taking any type of treatment. On the second control (day 7), the investigators, after the visit, give the patient the treatment box assigned according to the patient code assigned at the beginning of the study.

The study is double-blind, so the investigators do not know whether the patient has been assigned to the study group or to the placebo group.

### Type control

The control consists of a placebo without active ingredients with the same tri-layer appearance as the composition according to the invention.

### Masking techniques

The study samples are delivered in identical white boxes for both products (composition according to the invention and placebo). The labeling of the samples consists of a white label written in black ink, which includes study code and patient code). Also, expiration date and storage conditions should be included. Neither the physician nor the patient will know whether the box given/received at the visits corresponds to placebo or to the food supplement under study.

The Investigator's Manual includes the technical data sheets of both products (study samples and placebo).

### Subject selection

Patients over 18 years of age are to be included, with a diagnosis of insomnia according to the Sleep Test (modified test of the "Oviedo Sleep Questionnaire"), responded during the pre-assessment phase, and who are not under treatment for insomnia.

### Inclusion Criteria

- Older patients with diagnostic criteria of insomnia DSM-5 or ICSD-3 according to the Sleep Test (TS: modified from the "Oviedo Sleep Questionnaire").
- Patients with insomnia of the following type: insomnia of conciliation (> 30 minutes of sleep latency), maintenance insomnia (frequent awakenings or inability to fall asleep after an awakening) or mixed insomnia.
- Patients who are not taking food supplements or drugs for the treatment of insomnia.
- Patients who can understand the implications of the study and who demonstrate this by voluntarily signing the informed consent form.

### Exclusion Criteria

- Pregnant or breastfeeding women
- Patients with other sleep disorders: narcolepsy, obstructive apnea, circadian rhythm disorder, parasomnia.
- Patients with an active psychiatric disorder or cognitive impairment.
- Patients with serious illnesses that, in the physician's opinion, may interfere with the results of the study or make it inadvisable for them to participate in the study.
- Patients who do not give written consent.

### Number of subjects planned

The number of subjects to be enrolled in this study is 50 patients with insomnia, 25 in the study group and 25 in the placebo group.

### Sample size justification

The objective of this study is to demonstrate the ability of the composition according to the invention (Examples 1-9) to improve the quality and quantity of sleep as assessed by actigraphy and validated scales.

### Withdrawal or abandonment criteria

Subjects can withdraw from the study at their own request or by decision of the investigator for any of the following reasons:
- Voluntary withdrawal of the patient and revocation of consent.
- At the physician's discretion for safety reasons, protocol deviations, non-adherence, or for administrative reasons.
- Active intercurrent illness that may interfere with the conduct of the study.
- Occurrence of a Serious Adverse Event.

Patients who do not follow the study treatment regimen for at least the first 5 days and/or who do not attend the control visits will be considered to have dropped out of the study.

The reasons for withdrawal or abandonment of the study must be clearly documented in the CRD (End of Study section) if they are known.

Subjects who are excluded or drop out of the study for any of the above reasons are not to be replaced.

### Treatment under study

The treatment under study consists of a composition according to the invention (in a triple-layered tablet format) according to Examples 1-8 above.

The administration pattern will consist of taking 1 tablet per day of the product (composition of the invention or placebo) 30 minutes before going to bed.

### Placebo

The placebo will consist of triple-layered tablets, with the same appearance as the composition of the invention under study, but without active ingredients.

The administration regimen will consist of taking 1 tablet per day of the product (supplement or placebo) 30 minutes before going to bed.

### General considerations

The study samples will be delivered in white boxes with labels identifying the study and the patient, which will be identical for both products (composition of the invention and placebo) and the dosing regimen will be the same for both products, thus maintaining the double blind.

During the study, it will not be allowed to take any other pharmacological or non-pharmacological treatment for insomnia.

Data are obtained from patients' medical records, the physician-patient interview, the actygraph record and electronic questionnaires.

### Aim Variables

The main variables are obtained from the recording of the actigraphy recording, at two times basal (recording during the first week without treatment) and final (recording during the second week with treatment)
- Mean sleep latency during recording (LS)
- Total sleep time (TTS)
- Number of nocturnal awakenings
- Distribution of sleep-wake periods throughout the day (DSV)

### Secondary Variables

- Sociodemographic data (sex, age, pathologies)
- Time of evolution (weeks, months)
- Causes associated with insomnia (yes/no): stress/anxiety, depression, work schedules,...
- Symptoms secondary to insomnia (VAS scale from 0 to 10): tiredness, drowsiness, headache, concentration problems, ...
- Score on the sleep test
- Score on the Sleep Quality Scale (Pittsburgh or PSQI)
- Score on the Insomnia Severity Scale (ISI)
- Electronic Sleep Diary (ESD) score
- General quality of life and health status score (SF-12)
- Tolerability and safety of the product, assessed as the incidence of treatment-related adverse effects and adverse reactions
- Patient and physician opinion regarding the efficacy and tolerability of dietary supplement treatment, using 5-point Likert scales (0=very poor, 1=poor, 2=average, 3=good, 4=very good) at the end of the study
- Patient and physician satisfaction with dietary supplement treatment, 5-point Likert scales (0=very dissatisfied, 1=dissatisfied, 2=indifferent, 3=satisfied, 4=very satisfied) at the end of the study.

### Data collection

Patient data will be obtained from medical records, patient-physician interviews, chart records, and electronic questionnaires.

### Patients follow-up

The clinical follow-up of the patients is in 14 days. There are 3 on-site controls (initial, 7 days and 14 days). In addition, a preliminary assessment of the possible candidates for the study is made by completing a sleep test to confirm the diagnosis of insomnia before informing them about the study and obtaining informed consent.

**Pre-assessment of candidates.** Patients are identified from among those who have voluntarily and spontaneously entered the website of the Instituto del Sueño and completed the sleep test. Once those subjects who meet the diagnostic criteria for insomnia have been identified, who present with insomnia of conciliation, maintenance or mixed insomnia; and who meet all the inclusion/exclusion criteria; the physician contacts them to inform them about the study and propose their participation. If they accept, the physician sends them the informed consent form and will schedule an in-person visit.

**Visit 1 or initial visit (day 1).** Patients are included after confirming the inclusion and exclusion criteria and after obtaining the signature of the informed consent form.

The patients' demographic data (sex, age), personal history, insomnia history and typology (time of evolution, previous treatments, type of disorder, associated causes, secondary symptoms) are recorded and they are given access to the subjective perception of sleep (PSQI) and insomnia severity (ISI) questionnaires, and the general quality of life and health status questionnaire (SF-12). The physician gives the actigraphy to the patient and provide instructions on how to use it. The physician also provides access to the electronic sleep diary for the patient to complete each day. At the end of the visit, the physician schedules the patient for the next follow-up visit in 1 week.

**Day 7 (Subsequent follow-up visit in 1 week):** At this visit, the physician downloads the data from the electronic sleep diary and give it back to the patient for data recording during the following week. The physician also collects the first week's electronic sleep diary data for evaluation. The physician then provides the corresponding treatment (according to the assigned patient code).

**Day 14 (Final examination in 2 weeks):** On this visit the physician picks up the chart and record the last week's data. He/she also reviews and evaluate the data recorded in the electronic sleep diary. During the visit he/she records the data corresponding to therapeutic compliance and the assessment of symptoms associated with insomnia, reassessing the patient on the subjective perception of sleep (PSQI) and insomnia severity (ISI) questionnaires, recording the possible occurrence of adverse effects and events and the assessment of the degree of satisfaction with the treatment by the patient and him/her.

Data analysis are performed using the Statistical Analysis System (SAS), version 9.2.

### Study population

### Efficiency Population

The efficacy population consists of all patients who meet the inclusion/exclusion criteria and for whom efficacy data are available, i.e., for at least one of the primary endpoints of the primary endpoint in the study controls.

### Security population

All patients who have received at least one dose of the treatment under study.

### Descriptive statistics

A descriptive analysis is made of all the variables collected in the study stratified by groups and by visits. Continuous variables are described in terms of central tendency (Mean, SD, Min, P25, Median, P75, Max, 95%CI of Mean, Total, N Missing), categorical or ordinal variables will be described by frequencies(n) and percentages (%).

### Objectives analysis

Target analyses are performed for the efficacy population. An overall analysis and a group analysis (study group and placebo group) are performed.

### Main Objective

The main objective of the study is to evaluate the ability of the food supplement to improve the quality and duration of sleep by means of the actigraphy records. On the one hand, the main variables (mean sleep latency, total sleep time, distribution of sleep and wakefulness periods and number of nocturnal awakenings) is compared in all patients before treatment and during the treatment time and then the changes observed throughout the study with respect to the variables of the actigraphy recordings is compared between both groups.

### Secondary objectives

To evaluate the capacity of the food supplement to improve the self-perception of sleep quality and the intensity of insomnia, the scores obtained in the different scales is compared between the initial and final visit and between both groups of the study. Likewise, the evolution of the symptoms associated with insomnia in both groups and between both groups is studied.

The possible relationship between the variables of insomnia history and typology and the changes obtained in the actigraphy records and the different scales is studied.

### Safety Analysis

The safety analysis is performed with the safety population. All the analyses to be performed are descriptive in nature, with no statistical tests being performed in this analysis.

The variables to be described as safety variables are those related to:
- Adverse Events
- Abandonments and reasons for them

### Treatment of missing data

No formal imputation is performed for the different analyses, therefore all estimates is obtained using all available data (Available Data Only -ADO-)

### General aspects

This study is conducted in accordance with current regulations, the accepted international ethical standards of Good Clinical Practice (CPMP/ICH/135/95), the principles established in the latest version of the Declaration of Helsinki and the Biomedical Research Act 14/2007 of July 3, 2007.

Preferred embodiments E(n) of the present invention are the following:
E1. A composition comprising:
   - a mixture of active ingredients comprising or, alternatively, consisting of: a melatonin, a piperine, and a vitamin D,
      and the composition further comprises at least one additive and/or excipient of pharmaceutical or food grade.
E2. The composition according to E1, wherein the mixture further comprises a vitamin B6 in addition to said vitamin D; preferably, said vitamin D is a vitamin D3; more preferably, said mixture comprises a melatonin, a piperine, a vitamin D3, and a vitamin B6.
E3. The composition according to E1 or E2, wherein said mixture further comprises an extract of *Griffonia* (or *Griffonia simplicifolia*) or 5-hydroxytryptophan (5-HTP).
E4. The composition according to any one of E1-E3, wherein said mixture further comprises at least one active ingredient selected in the group consisting of: an extract of *Valeriana officinalis,* extract of *Melissa officinalis,* extract of *Passiflora* and extract of *Eschscholzia californica*;
   preferably said mixture comprises or, alternatively, consists of: melatonin, piperine, vitamin D3, vitamin B6, extract of *Griffonia,* extract of *Passiflora,* extract of *Eschscholzia californica,* extract of *Valeriana officinalis,* and extract of *Melissa officinalis.*
E5. A controlled-release formulation for oral administration comprising:
   - a fast release composition comprising as active ingredients melatonin, piperine, and vitamin D;
   - a medium release composition comprising as active ingredients an extract of *Valeriana officinalis* and/or an extract of *Melissa officinalis;* and
   - a prolonged release composition comprising as active ingredient an extract of *Griffonia* (or *Griffonia simplicifolia*) or 5-hydroxytryptophan (5-HTP),
      wherein the fast release composition is degraded *in vivo* within a period of time comprised from about 0 minute to about 20 minutes, preferably from about 0 minute to about 5 minutes, after the administration;
      wherein the medium release composition is degraded *in vivo* within a period of time comprised from about 0 minute to about 2.5 hours, preferably from about 0 minute to 2 hours, after the administration;
      wherein the prolonged release composition is degraded *in vivo* within a period of time comprised from about 1.5 hours to about 5 hours, preferably from 2 hours to 4 hours, after the administration;
      preferably, wherein the formulation is a monolithic system, the compositions are compressed composition, and the compressed compositions are in stacked arrangement.
E6. The formulation according to E1 or E2, wherein the fast release composition further comprises as active ingredient a vitamin B6;
   preferably, wherein vitamin D is vitamin D3;
   preferably, wherein the fast release composition comprises as active ingredients melatonin, piperine, vitamin D3, and vitamin B6.
E7. The formulation according to E1, wherein the prolonged release composition further comprises as active ingredients an extract of *Passiflora* and/or an extract of *Eschscholzia californica*;
   preferably, an extract of *Passiflora incarnata* and an extract of *Eschscholzia californica.*
E8. The formulation according to any one of E1-E3, wherein:
   - as active ingredients, the fast release composition comprises or, alternatively, consists of melatonin, piperine, vitamin D, preferably vitamin D3, and vitamin B6,
   - as active ingredients, the medium release composition comprises or, alternatively, consists of extract of *Valeriana officinalis* and extract of *Melissa officinalis,* and
   - as active ingredients, the prolonged release composition comprises or, alternatively, consists of extract of *Griffonia* or 5-hydroxytryptophan, and extract of *Passiflora incarnata* and extract of *Eschscholzia californica.*
E9. The formulation according to any one of E1-E8, wherein:
   - the fast release composition comprises melatonin in a rate of 0.5 mg to 20 mg, piperine in a rate of 0.5 mg to 50 mg, vitamin D3 in a rate of 0.5 µg to 50 µg and, optionally, vitamin B6 in a rate of 0.1 mg to 20 mg;
      preferably melatonin in a rate of 1 mg to 5 mg, piperine in a rate of 2.5 mg to 10 mg, vitamin D3 in a rate of 1 µg to 10 µg, and, optionally, vitamin B6 in a rate of 0.5 mg to 5 mg;
   - the medium release composition comprises extract of *Valeriana officinalis* in a rate of 10 mg to 500 mg and/or extract of *Melissa officinalis* in a rate of 10 mg to 500 mg,
      preferably extract of *Valeriana officinalis* in a rate of 50 mg to 200 mg and/or extract of *Melissa officinalis* in a rate of 50 mg to 200 mg; and
   - the prolonged release composition comprises extract of *Griffonia* (or *Griffonia simplicifolia*) or 5-hydroxytryptophan in a rate of 1 mg to 300 mg, preferably in a rate of 10 mg to 60 mg, and, optionally, extract of *Passiflora incarnata* in a rate of 10 mg to 500 mg and/or extract of *Eschscholzia californica* in a rate of 10 mg to 500 mg, preferably extract of *Passiflora incarnata* in a rate of 50 mg to 200 mg and/or extract of *Eschscholzia californica* in a rate of 50 mg to 200 mg.
E10. The composition and the fast-release formulation according to any one of E1-E9 for use in a method of treatment of circadian rhythm sleep disorders and symptoms associated thereof;
   preferably, wherein the circadian rhythm sleep disorders and associated symptoms thereof are selected in the group consisting of: insomnia, inability to fall asleep, difficulty falling asleep, early awakening, and interrupted sleep, excessive daytime sleepiness, symptoms of shift work disorder, jet lag disorder (JLD) and symptoms thereof, dream disorders, dream disorders in Post-Traumatic Stress syndrome.

Further preferred embodiments F(n) of the present invention are the following:
F1. A composition comprising:
   - a mixture of active ingredients comprising or, alternatively, consisting of: a melatonin, a piperine and/or capsaicin or a capsaicin analogue (preferably phenylcapsaicin), and a vitamin D,
      and the composition further comprises at least one additive and/or excipient of pharmaceutical or food grade.
F2. The composition according to F1, wherein the mixture further comprises a vitamin B6, and optionally Zea *mays* extract and/or capsaicin or a capsaicin analogue (preferably phenylcapsaicin), in addition to said vitamin D; preferably, said vitamin D is a vitamin D3; more preferably, said mixture comprises a melatonin, a piperine, a vitamin D3, a vitamin B6 and, optionally, Zea *mays* extract and/or capsaicin or a capsaicin analogue (preferably phenylcapsaicin).
F3. The composition according to F1 or F2, wherein said mixture further comprises an extract of *Griffonia* (or *Griffonia simplicifolia*) or 5-hydroxytryptophan (5-HTP) or Zea *mays* extract or Gaba.
F4. The composition according to any one of F1-F3, wherein said mixture further comprises at least one active ingredient selected in the group consisting of: an extract of *Valeriana officinalis,* extract of *Melissa officinalis,* extract of *Passiflora,* extract of *Eschscholzia californica,* Zea *mays* extract, and gamma-aminobutyric acid (GABA) or milk protein hydrolized, preferably Lactium^{®} having the following characteristics:
   - A total moisture from 3% to 8%, preferably 4.5%;
   - A total protein from 16% to 90%, preferably 80%;
   - A total fat from 0.1 % to 1%, preferably 0.5%;
   - A total carbohydrate from 0.05% to 0.5%, preferably 0.3%.
F5. The composition according to F4, wherein said mixture comprises or, alternatively, consists of: melatonin, piperine, vitamin D3, vitamin B6, extract of *Griffonia,* extract of *Passiflora,* extract of *Eschscholzia californica,* extract of *Valeriana officinalis,* and extract of *Melissa officinalis.*
F6. The composition according to F4, wherein said mixture comprises or, alternatively, consists of: melatonin, piperine, vitamin D3, vitamin B6, Zea *mays* extract, extract of *Valeriana officinalis,* and extract of *Melissa officinalis.*
F7. The composition according to F4, wherein said mixture comprises or, alternatively, consists of: melatonin, Zea *mays* extract, piperine, vitamin D3, vitamin B6, extract of *Passiflora,* extract of *Eschscholzia californica,* extract of *Valeriana officinalis,* and extract of *Melissa officinalis.*
F8. The composition according to F4, wherein said mixture comprises or, alternatively, consists of: melatonin, piperine, vitamin D3, vitamin B6, gamma-aminobutyric acid (GABA) or a salt or an ester thereof, extract of *Passiflora,* extract of *Eschscholzia californica,* extract of *Valeriana officinalis,* and extract of *Melissa officinalis.*
F9. A controlled-release formulation for oral administration comprising:
   - a fast release composition comprising as active ingredients melatonin, piperine, and vitamin D;
   - a medium release composition comprising as active ingredients an extract of *Valeriana officinalis* and/or an extract of *Melissa officinalis;* and
   - a prolonged release composition comprising as active ingredient an extract of *Griffonia* (or *Griffonia simplicifolia*) or 5-hydroxytryptophan (5-HTP), or Zea *mays* extract, or gamma-aminobutyric acid (GABA) or a salt or an ester thereof,
      wherein the fast release composition is degraded *in vivo* within a period of time comprised from about 0 minute to about 20 minutes, preferably from about 0 minute to about 5 minutes, after the administration;
      wherein the medium release composition is degraded *in vivo* within a period of time comprised from about 0 minute to about 2.5 hours, preferably from about 0 minute to 2 hours, after the administration;
      wherein the prolonged release composition is degraded *in vivo* within a period of time comprised from about 1.5 hours to about 5 hours, preferably from 2 hours to 4 hours, after the administration;
      preferably, wherein the formulation is a monolithic system, the compositions are compressed composition, and the compressed compositions are in stacked arrangement.
F10. The formulation according to F9, wherein the fast release composition further comprises as active ingredient a vitamin B6, and optionally Zea *mays* extract and/or capsaicin or a capsaicin analogue (preferably phenylcapsaicin);
   preferably, wherein vitamin D is vitamin D3;
   preferably, wherein the fast release composition comprises as active ingredients melatonin, piperine, vitamin D3, vitamin B6 and, optionally, Zea *mays* extract and/or capsaicin or a capsaicin analogue (preferably phenylcapsaicin).
F11. The formulation according to F9, wherein the prolonged release composition further comprises as active ingredients an extract of *Passiflora* and/or an extract of *Eschscholzia californica* and/or *Zea mays* extract and/or gamma-aminobutyric acid (GABA) or a salt or an ester thereof;
   preferably, an extract of *Passiflora incarnata* and an extract of *Eschscholzia californica,* or *Zea mays* extract, or *Passiflora incarnata* and an extract of *Eschscholzia californica* and *Zea mays* extract, or *Passiflora incarnata* and an extract of *Eschscholzia californica* and gamma-aminobutyric acid (GABA) or a salt or an ester thereof.
F12. The formulation according to any one of F9-F11, wherein:
   - as active ingredients, the fast release composition comprises or, alternatively, consists of melatonin, piperine, vitamin D, preferably vitamin D3, vitamin B6, and optionally *Zea mays* extract and/or capsaicin or a capsaicin analogue (preferably phenylcapsaicin);
   - as active ingredients, the medium release composition comprises or, alternatively, consists of extract of *Valeriana officinalis* and extract of *Melissa officinalis,* and
   - as active ingredients, the prolonged release composition comprises or, alternatively, consists of extract of *Griffonia* or 5-hydroxytryptophan, and extract of *Passiflora incarnata* and extract of *Eschscholzia californica,* or *Zea mays* extract, or *Zea mays* extract and extract of *Passiflora incarnata* and extract of *Eschscholzia californica,* or gamma-aminobutyric acid (GABA) or a salt or an ester thereof.
F13. The formulation according to any one of F9-F12, wherein:
   - the fast release composition comprises melatonin in a rate of 0.5 mg to 20 mg, piperine in a rate of 0.5 mg to 50 mg, vitamin D3 in a rate of 0.5 µg to 50 µg and, optionally, vitamin B6 in a rate of 0.1 mg to 20 mg and, optionally, Zea *mays* extract in a rate of 50 mg to 300 mg, and/or, optionally, capsaicin or a capsaicin analogue (preferably phenylcapsaicin) in a rate from 0,5 mg to 300 mg, preferably from 10 mg to 200 mg, more preferably from 50 mg to 100 mg.;
      preferably melatonin in a rate of 1 mg to 5 mg, piperine in a rate of 2.5 mg to 10 mg, vitamin D3 in a rate of 1 µg to 10 µg, and, optionally, vitamin B6 in a rate of 0.5 mg to 5 mg and, optionally, *Zea mays* extract in a rate of 100 mg to 200 mg, and/or, optionally, capsaicin or a capsaicin analogue (preferably phenylcapsaicin) in a rate from 0,5 mg to 300 mg, preferably from 10 mg to 200 mg, more preferably from 50 mg to 100 mg.;
   - the medium release composition comprises extract of *Valeriana officinalis* in a rate of 10 mg to 500 mg and/or extract of *Melissa officinalis* in a rate of 10 mg to 500 mg,
      preferably extract of *Valeriana officinalis* in a rate of 50 mg to 200 mg and/or extract of *Melissa officinalis* in a rate of 50 mg to 200 mg; and
   - the prolonged release composition comprises:
      (i) extract of *Griffonia* (or *Griffonia simplicifolia*) or 5-hydroxytryptophan in a rate of 1 mg to 300 mg, preferably in a rate of 10 mg to 60 mg, and, optionally, extract of *Passiflora incarnata* in a rate of 10 mg to 500 mg and/or extract of *Eschscholzia californica* in a rate of 10 mg to 500 mg, preferably extract of *Passiflora incarnata* in a rate of 50 mg to 200 mg and/or extract of *Eschscholzia californica* in a rate of 50 mg to 200 mg; or
      (ii) *Zea mays* extract in a rate of 50 mg to 250 mg, preferably in a rate of 70 mg to 200 mg, and, optionally, extract of *Passiflora incarnata* in a rate of 10 mg to 500 mg and/or extract of *Eschscholzia californica* in a rate of 10 mg to 500 mg, preferably extract of *Passiflora incarnata* in a rate of 50 mg to 200 mg and/or extract of *Eschscholzia californica* in a rate of 50 mg to 200 mg; or
      (iii) gamma-aminobutyric acid (GABA) or a salt or an ester thereof in a rate of 1 mg to 300 mg, preferably in a rate of 50 mg to 200 mg, and, optionally, extract of *Passiflora incarnata* in a rate of 10 mg to 500 mg and/or extract of *Eschscholzia californica* in a rate of 10 mg to 500 mg, preferably extract of *Passiflora incarnata* in a rate of 50 mg to 200 mg and/or extract of *Eschscholzia californica* in a rate of 50 mg to 200 mg.
F14. The composition and the fast-release formulation according to any one of F1-F13 for use in a method of treatment of circadian rhythm sleep disorders and symptoms associated thereof;
   preferably, wherein the circadian rhythm sleep disorders and associated symptoms thereof are selected in the group consisting of: insomnia, inability to fall asleep, difficulty falling asleep, early awakening, and interrupted sleep, excessive daytime sleepiness, symptoms of shift work disorder, jet lag disorder (JLD) and symptoms thereof, dream disorders, dream disorders in Post-Traumatic Stress syndrome.

## Claims

1. A composition comprising:
- a mixture of active ingredients comprising or, alternatively, consisting of: a melatonin, a piperine and/or a capsaicin or a capsaicin analogue, and a vitamin D,
and the composition further comprises at least one additive and/or excipient of pharmaceutical or food grade.

2. The composition according to claim 1, wherein said composition comprising:
- a mixture of active ingredients comprising or, alternatively, consisting of: a melatonin, a piperine and a vitamin D, or
- a mixture of active ingredients comprising or, alternatively, consisting of: a melatonin, a capsaicin or a capsaicin analogue, and a vitamin D.

3. The composition according to claim 1 or 2, wherein the mixture further comprises a vitamin B6, and optionally *Zea mays* extract and/or capsaicin or a capsaicin analogue (preferably phenylcapsaicin), in addition to said vitamin D; preferably, said vitamin D is a vitamin D3; more preferably, said mixture comprises a melatonin, a piperine, a vitamin D3, a vitamin B6 and, optionally, *Zea mays* extract and/or capsaicin or a capsaicin analogue (preferably phenylcapsaicin).

4. The composition according to anyone claims 1 or 2 or 3, wherein said mixture further comprises an extract of *Griffonia* (or *Griffonia simplicifolia*) or 5-hydroxytryptophan (5-HTP) or *Zea mays* extract or Gaba.

5. The composition according to any one of claims 1-4, wherein said mixture further comprises at least one active ingredient selected in the group consisting of: an extract of *Valeriana officinalis,* extract of *Melissa officinalis,* extract of *Passiflora,* extract of *Eschscholzia californica, Zea mays* extract, and gamma-aminobutyric acid (GABA) or milk protein hydrolized, preferably Lactium^{®} having the following characteristics:
- A total moisture from 3% to 8%, preferably 4.5%;
- A total protein from 16% to 90%, preferably 80%;
- A total fat from 0.1 % to 1%, preferably 0.5%;
- A total carbohydrate from 0.05% to 0.5%, preferably 0.3%.

6. The composition according to claim 5, wherein said mixture comprises or, alternatively, consists of: melatonin, piperine, vitamin D3, vitamin B6, extract of *Griffonia,* extract of *Passiflora,* extract of *Eschscholzia californica,* extract of *Valeriana officinalis,* and extract of *Melissa officinalis.*

7. The composition according to claim 5, wherein said mixture comprises or, alternatively, consists of: melatonin, piperine, vitamin D3, vitamin B6, *Zea mays* extract, extract of *Valeriana officinalis,* and extract of *Melissa officinalis.*

8. The composition according to claim 5, wherein said mixture comprises or, alternatively, consists of: melatonin, *Zea mays* extract, piperine, vitamin D3, vitamin B6, extract of *Passiflora,* extract of *Eschscholzia californica,* extract of *Valeriana officinalis,* and extract of *Melissa officinalis.*

9. The composition according to claim 5, wherein said mixture comprises or, alternatively, consists of: melatonin, piperine, vitamin D3, vitamin B6, gamma-aminobutyric acid (GABA) or a salt or an ester thereof, extract of *Passiflora,* extract of *Eschscholzia californica,* extract of *Valeriana officinalis,* and extract of *Melissa officinalis.*

10. A controlled-release formulation for oral administration comprising:
- a fast release composition comprising as active ingredients melatonin, piperine, and vitamin D;
- a medium release composition comprising as active ingredients an extract of *Valeriana officinalis* and/or an extract of *Melissa officinalis;* and
- a prolonged release composition comprising as active ingredient an extract of *Griffonia* (or *Griffonia simplicifolia*) or 5-hydroxytryptophan (5-HTP), or *Zea mays* extract, or gamma-aminobutyric acid (GABA) or a salt or an ester thereof,
wherein the fast release composition is degraded *in vivo* within a period of time comprised from about 0 minute to about 20 minutes, preferably from about 0 minute to about 5 minutes, after the administration; wherein the medium release composition is degraded *in vivo* within a period of time comprised from about 0 minute to about 2.5 hours, preferably from about 0 minute to 2 hours, after the administration;
wherein the prolonged release composition is degraded *in vivo* within a period of time comprised from about 1.5 hours to about 5 hours, preferably from 2 hours to 4 hours, after the administration;
preferably, wherein the formulation is a monolithic system, the compositions are compressed composition, and the compressed compositions are in stacked arrangement.

11. The formulation according to claim 10, wherein the fast release composition further comprises as active ingredient a vitamin B6, and optionally *Zea mays* extract and/or capsaicin or a capsaicin analogue (preferably phenylcapsaicin);
preferably, wherein vitamin D is vitamin D3;
preferably, wherein the fast release composition comprises as active ingredients melatonin, piperine, vitamin D3, vitamin B6 and, optionally, *Zea mays* extract and/or capsaicin or a capsaicin analogue (preferably phenylcapsaicin).

12. The formulation according to claim 9, wherein the prolonged release composition further comprises as active ingredients an extract of *Passiflora* and/or an extract of *Eschscholzia californica* and/or *Zea mays* extract and/or gamma-aminobutyric acid (GABA) or a salt or an ester thereof;
preferably, an extract of *Passiflora incarnata* and an extract of *Eschscholzia californica,* or *Zea mays* extract, or *Passiflora incarnata* and an extract of *Eschscholzia californica* and *Zea mays* extract, or *Passiflora incarnata* and an extract of *Eschscholzia californica* and gamma-aminobutyric acid (GABA) or a salt or an ester thereof.

13. The formulation according to any one of claims 10-12, wherein:
- as active ingredients, the fast release composition comprises or, alternatively, consists of melatonin, piperine, vitamin D, preferably vitamin D3, vitamin B6, and optionally *Zea mays* extract and/or capsaicin or a capsaicin analogue (preferably phenylcapsaicin);
- as active ingredients, the medium release composition comprises or, alternatively, consists of extract of *Valeriana officinalis* and extract of *Melissa officinalis,* and
- as active ingredients, the prolonged release composition comprises or, alternatively, consists of extract of *Griffonia* or 5-hydroxytryptophan, and extract of *Passiflora incarnata* and extract of *Eschscholzia californica,* or *Zea mays* extract, or *Zea mays* extract and extract of *Passiflora incarnata* and extract of *Eschscholzia californica,* or gamma-aminobutyric acid (GABA) or a salt or an ester thereof.

14. The formulation according to any one of claims 10-13, wherein:
- the fast release composition comprises melatonin in a rate of 0.5 mg to 20 mg, piperine in a rate of 0.5 mg to 50 mg, vitamin D3 in a rate of 0.5 µg to 50 µg and, optionally, vitamin B6 in a rate of 0.1 mg to 20 mg and, optionally, *Zea mays* extract in a rate of 50 mg to 300 mg, and/or, optionally, capsaicin or a capsaicin analogue (preferably phenylcapsaicin) in a rate of 10 mg to 300 mg;
preferably melatonin in a rate of 1 mg to 5 mg, piperine in a rate of 2.5 mg to 10 mg, vitamin D3 in a rate of 1 µg to 10 µg, and, optionally, vitamin B6 in a rate of 0.5 mg to 5 mg and, optionally, *Zea mays* extract in a rate of 100 mg to 200 mg, and/or, optionally, capsaicin or a capsaicin analogue (preferably phenylcapsaicin) in a rate of 50 mg to 200 mg;
- the medium release composition comprises extract of *Valeriana officinalis* in a rate of 10 mg to 500 mg and/or extract of *Melissa officinalis* in a rate of 10 mg to 500 mg,
preferably extract of *Valeriana officinalis* in a rate of 50 mg to 200 mg and/or extract of *Melissa officinalis* in a rate of 50 mg to 200 mg; and
- the prolonged release composition comprises:
(i) extract of *Griffonia* (or *Griffonia simplicifolia*) or 5-hydroxytryptophan in a rate of 1 mg to 300 mg, preferably in a rate of 10 mg to 60 mg, and, optionally, extract of *Passiflora incarnata* in a rate of 10 mg to 500 mg and/or extract of *Eschscholzia californica* in a rate of 10 mg to 500 mg, preferably extract of *Passiflora incarnata* in a rate of 50 mg to 200 mg and/or extract of *Eschscholzia californica* in a rate of 50 mg to 200 mg; or
(ii) *Zea mays* extract in a rate of 50 mg to 250 mg, preferably in a rate of 70 mg to 200 mg, and, optionally, extract of *Passiflora incarnata* in a rate of 10 mg to 500 mg and/or extract of *Eschscholzia californica* in a rate of 10 mg to 500 mg, preferably extract of *Passiflora incarnata* in a rate of 50 mg to 200 mg and/or extract of *Eschscholzia californica* in a rate of 50 mg to 200 mg; or
(iii) gamma-aminobutyric acid (GABA) or a salt or an ester thereof in a rate of 1 mg to 300 mg, preferably in a rate of 50 mg to 200 mg, and, optionally, extract of *Passiflora incarnata* in a rate of 10 mg to 500 mg and/or extract of *Eschscholzia californica* in a rate of 10 mg to 500 mg, preferably extract of *Passiflora incarnata* in a rate of 50 mg to 200 mg and/or extract of *Eschscholzia californica* in a rate of 50 mg to 200 mg.

15. The composition and the fast-release formulation according to any one of claims 1-14 for use in a method of treatment of circadian rhythm sleep disorders and symptoms associated thereof;
preferably, wherein the circadian rhythm sleep disorders and associated symptoms thereof are selected in the group consisting of: insomnia, inability to fall asleep, difficulty falling asleep, early awakening, and interrupted sleep, excessive daytime sleepiness, symptoms of shift work disorder, jet lag disorder (JLD) and symptoms thereof, dream disorders, dream disorders in Post-Traumatic Stress syndrome.
